(19) Europäisches Patentamt
European Patent Office
Office européen des brevets

(11) Numéro de publication : **0 058 097**
**B1**

(12) **FASCICULE DE BREVET EUROPÉEN**

(45) Date de publication du fascicule du brevet : **25.07.84**

(51) Int. Cl.³ : **C 07 J 5/00**, C 07 J 41/00

(21) Numéro de dépôt : **82400026.9**

(22) Date de dépôt : **08.01.82**

(54) **Nouveau procédé de préparation de 17alpha-hydroxy 17bêta-hydroxyacétyl stéroides et produits intermédiaires correspondants obtenus.**

(30) Priorité : **29.01.81 FR 8101688**

(43) Date de publication de la demande : **18.08.82 Bulletin 82/33**

(45) Mention de la délivrance du brevet : **25.07.84 Bulletin 84/30**

(84) Etats contractants désignés : **AT CH DE FR GB IT LI NL SE**

(56) Documents cités :
**EP-A- 0 023 856**
**JOURNAL OF THE CHEMICAL SOCIETY, Perkin Transactions I. 1975 LONDRES (GB) R.B. BOAR et al.: "A new and convenient synthesis of the 17alpha, 21-diacetoxy-20 oxopregnane side-chain" pages 1242-1244**

(73) Titulaire : **ROUSSEL-UCLAF**
**35, boulevard des Invalides**
**F-75007 Paris (FR)**

(72) Inventeur : **Nedelec, Lucien**
**45, boulevard de l'Ouest**
**F-93340 Le Raincy (FR)**
Inventeur : **Torelli, Vesperto**
**5, rue de la Convention**
**F-94700 Maisons Alfort (FR)**
Inventeur : **Hardy, Michel**
**No 3, Domaine du Château Gaillard**
**F-94700 Maisons Alfort (FR)**

(74) Mandataire : **Bourgouin, André et al**
**ROUSSEL-UCLAF 111, route de Noisy Boîte postale no 9**
**F-93230 Romainville (FR)**

EP 0 058 097 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un procédé de préparation de 17α-hydroxy 17β-hydroxyacétyl stéroïdes et des produits intermédiaires correspondants.

La présente invention a pour objet un procédé de préparation de produits de formule (I)

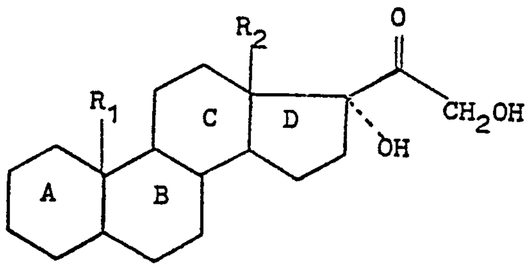

(I)

dans laquelle $R_1$ représente un atome d'hydrogène, un radical alkyle renfermant de 1 à 4 atomes de carbone substitué éventuellement par une fonction oxygénée ou azotée ou par un atome d'halogène ou $R_1$ représente un radical alkényle ou alkynyle renfermant de 2 à 4 atomes de carbone, $R_2$ représente un radical alkyle renfermant de 1 à 4 atomes de carbone, les noyaux A, B, C, D portent éventuellement une ou plusieurs doubles liaisons et sont éventuellement substitués par une ou plusieurs fonctions hydroxyle ou cétone, par un ou plusieurs atomes d'halogène, par un ou plusieurs radicaux alkyle ou alkyloxy renfermant de 1 à 4 atomes de carbone ou par un ou plusieurs radicaux alkényle ou alkynyle renfermant de 2 à 4 atomes de carbone, caractérisé en ce que l'on traite un produit de formule (II)

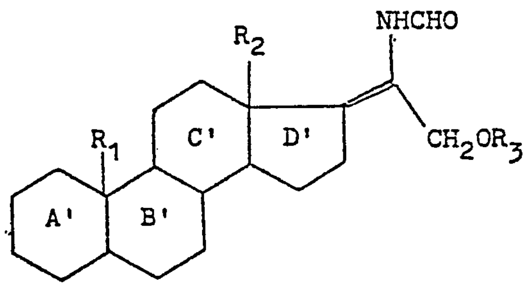

(II)

dans laquelle $R_1$ et $R_2$ ont la signification précédente et les noyaux A', B', C' et D' ont les mêmes valeurs que précédemment décrite pour A, B, C, D avec la possibilité supplémentaire d'être substitués par des fonctions hydroxyle ou cétone protégées et $R_3$ représente un atome d'hydrogène ou $R'_3$, $R'_3$ représentant un groupement protecteur du radical hydroxyle, par un réactif d'introduction du radical acétoxyle, pour obtenir un produit de formule (III) :

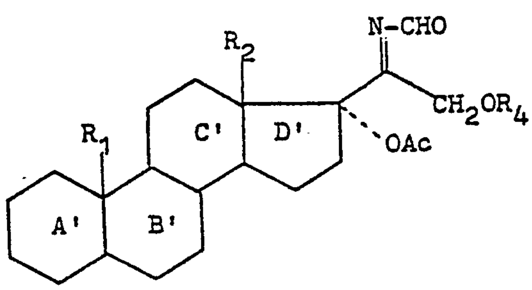

(III)

dans laquelle $R_4$ représente $R'_3$ ou un radical acétyle et Ac représente un radical acétyle, produit que l'on traite par un agent d'hydrolyse, pour obtenir un produit de formule (IV) :

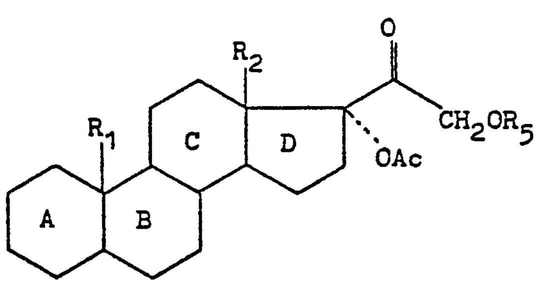

(IV)

dans laquelle $R_1$, $R_2$, Ac, A, B, C et D ont la signification précédente et $R_5$ représente un atome

d'hydrogène, un groupement protecteur du radical hydroxyle non facilement hydrolysable ou un radical acétyle, produit que l'on traite de manière à éliminer le radical acétyle en 17α et le groupement $R_5$ lorsque celui-ci ne représente pas un atome d'hydrogène, pour obtenir un produit de formule (I).

Il doit être noté que le produit de formule (II) peut être sous la forme 20E, 20Z ou sous la forme d'un mélange 20E et 20Z.

Parmi les valeurs de $R_1$, on peut citer notamment les radicaux méthyle, éthyle, propyle et butyle linéaires ou ramifiés.

On peut également citer, parmi les radicaux alkyles substitués par une fonction oxygénée, les radicaux hydroxyméthyle, hydroxyéthyle, formyle ou acétyle.

Lorsque $R_1$ représente un radical alkyle substitué par une fonction azotée, il s'agit de préférence du radical cyano ou du radical aminométhyle ou aminoéthyle.

Lorsque $R_1$ représente un radical alkyle substitué par un halogène, il s'agit de préférence d'un radical $—CH_2Hal$, dans lequel Hal représente un atome d'halogène, comme par exemple un atome de chlore, de fluor ou de brome.

Lorsque $R_1$ représente un radical alkényle, il s'agit de préférence du radical vinyle ou allyle.

Lorsque $R_1$ représente un radical alkynyle, il s'agit de préférence du radical éthynyle.

Le substituant $R_2$ peut représenter un radical méthyle, éthyle, propyle ou butyle mais représente de préférence un radical méthyle ou éthyle.

Lorsque les noyaux A, B, C et D portent une ou plusieurs doubles liaisons, il s'agit de préférence de doubles liaisons en 1(2), 4(5), 5(6) ou 9(11) ou d'un système de doubles liaisons conjuguées en 3(4) et 5(6) ou en 4(5) et 6(7) ou d'un système aromatique de trois doubles liaisons 1, 3, 5 ou d'un système de trois doubles liaisons 1(2), 4(5), 6(7).

Lorsque les cycles A, B, C et D sont substitués par une ou plusieurs fonctions hydroxyles, il s'agit de préférence d'une fonction hydroxyle en 3 ou en 11.

Lorsque les cycles A, B, C et D sont substitués par une ou plusieurs fonctions cétones, il s'agit de préférence d'une fonction cétone en 3 ou en 11.

Lorsque les cycles A, B, C et D sont substitués par un ou plusieurs atomes d'halogènes, il s'agit de préférence d'un atome de fluor, de chlore ou de brome, en position 6 ou 9α par exemple.

Lorsque les cycles A, B, C et D sont substitués par un ou plusieurs radicaux alkyles, il s'agit de préférence du radical méthyle ou éthyle en 2, 6, 7, en 16α ou en 16β.

Lorsque les cycles A, B, C et D sont substitués par un ou plusieurs radicaux alkyloxy, il s'agit de préférence d'un radical méthoxy ou éthoxy en 3 ou 11β.

Lorsque les cycles A, B, C et D sont substitués par un ou plusieurs radicaux alkényles, il s'agit de préférence du radical vinyle ou allyle en position 11β par exemple.

Lorsque les cycles A, B, C et D sont substitués par un ou plusieurs radicaux alkynyles, il s'agit de préférence du radical éthynyle en position 11β par exemple.

Lorsque les cycles A', B', C', D' comportent des groupements hydroxyles ou cétoniques protégés, ces groupements se trouvent de préférence en position 3 ou 11.

Les groupements hydroxyles peuvent être protégés selon les modes habituels connus dans la littérature. On peut par exemple citer les groupements acétonides, les carbonates cycliques, les orthoesters, les sulfites cycliques, l'éther formé avec le tétrahydropyrannyle, le groupement trityle, benzyle.

Les groupements cétoniques peuvent de même être protégés par les groupements protecteurs classiques tels que les cétals, plus spécialement l'éthylène cétal, les thiocétals, les hémithiocétals, les éthers d'énols, les acétates d'énols, les énamines, les oximes.

On préfère cependant les groupements cétals et spécialement l'éthylène cétal pour protéger les groupements cétoniques.

C'est ainsi que les produits 3,3-éthylène dioxy $\Delta_5$ ou 3,3-éthylène dioxy 5α-hydroxy sont préférés.

Parmi les produits de formule (II), on utilise de préférence les produits dans lesquels $R_3$ représente $R'_3$ c'est-à-dire un groupement protecteur du radical hydroxyle.

On peut utiliser l'un des groupements protecteurs connus dans la littérature tels que ceux indiqués ci-dessus.

On utilise de préférence, cependant, un groupement acyle tel que acétyle.

Lorsque l'on utilise comme produit de départ un produit de formule (II) dans laquelle $R_3$ représente un atome d'hydrogène, le radical hydroxyle en position 20 se trouve acétylé lors de l'action du réactif d'introduction du radical acétoxyle.

Bien entendu, le but principal de l'action de ce réactif est l'introduction d'un groupement acétoxyle en 17α. Le réactif que l'on utilise de préférence est le tétracétate de plomb ou l'iodosobenzène diacétate.

L'agent d'hydrolyse avec lequel on traite le produit de formule (III) est de préférence un agent d'hydrolyse acide tel que l'acide chlorhydrique, sulfurique, acétique ou trifluoroacétique.

Ce traitement a pour but de convertir la fonction formylimino en fonction cétone. De manière générale ce traitement a également pour conséquence de débloquer les fonctions hydroxyles ou cétoniques protégées de la molécule, c'est ainsi qu'un groupement cétal sera éliminé de cette manière pour redonner le groupement cétonique libre. De même lorsque $R_4$ représente un groupement protecteur facilement éliminable par hydrolyse, ce groupement est éliminé ; ceci est le cas par exemple lorsque $R_4$

représente un groupement tétrahydropyrannyle.

Par contre, lorsque $R_4$ représente un groupement protecteur non facilement éliminable par hydrolyse tel qu'un radical acyle, de préférence acétyle, ce radical est maintenu.

Le ou les traitements des produits de formule (IV) ont pour but d'éliminer le radical acétyle en position 17α et le radical $R_5$ lorsque celui-ci ne représente pas un atome d'hydrogène mais un radical non facilement éliminable par hydrolyse tel qu'un acyle, de préférence un acétyle.

Le traitement est de préférence une saponification réalisée dans les conditions usuelles. On utilise alors d'abord une base telle que la soude, la potasse, la baryte ou un sel tel que les carbonates de métaux alcalins. De manière à isoler plus facilement le produit obtenu, on acidifie ensuite de préférence le milieu réactionnel par un acide tel que l'acide chlorhydrique, sulfurique ou acétique.

Selon la valeur du radical $R_5$, la réaction précédente peut être complétée par une autre réaction de manière à éliminer ce radical. Cette réaction peut, par exemple être une hydrogénolyse réalisée dans les conditions usuelles.

Il est entendu que les étapes du procédé que la demanderesse entend plus spécialement réserver sont les étapes d'introduction du radical acétoxyle et d'hydrolyse de la fonction formylimino. Plus particulièrement, l'étape d'introduction du radical acétoxyle est l'étape essentielle du procédé objet de la présente demande.

La présente invention a plus spécialement pour objet un procédé tel que défini ci-dessus, caractérisé en ce que l'on utilise comme produits de départ des produits de formule (II) dans laquelle $R_2$ représente un radical méthyle et $R_1$ représente un atome d'hydrogène ou un radical méthyle ou éthynyle.

La présente invention concerne plus spécialement un procédé de préparation des produits de formule (I')

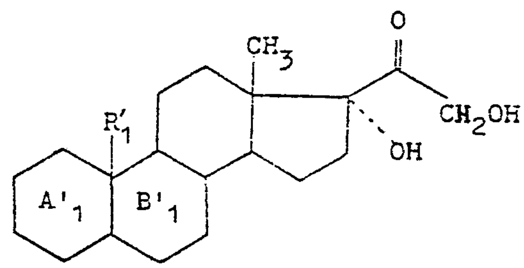

(I')

dans laquelle $R'_1$, $A'_1$ et $B'_1$ sont tels que les noyaux $A'_1$ et $B'_1$ sont choisis dans le groupe formé par les systèmes suivants :

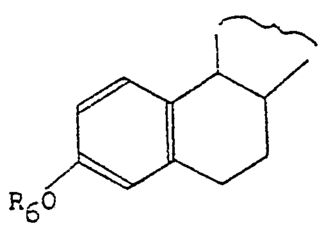

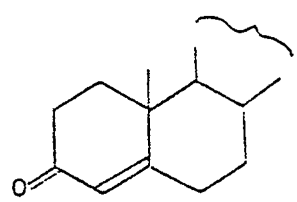

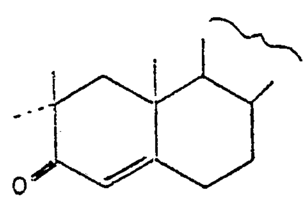

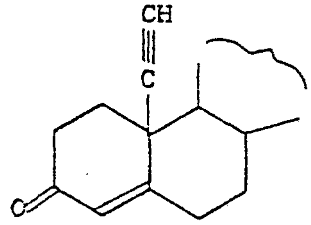

dans lequel $R_6$ représente un radical alkyle ayant de 1 à 8 atomes de carbone ou un radical aralkyle ayant de 7 à 12 atomes de carbone, caractérisé en ce que l'on traite un produit de formule (II')

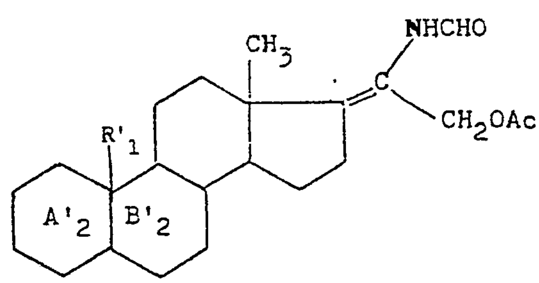

(II')

dans laquelle $R'_1$ a la signification indiquée précédemment, $A_c$ représente un radical acétyle et les noyaux $A'_1$ et $B'_2$ sont choisis dans le groupe formé par les systèmes suivants :

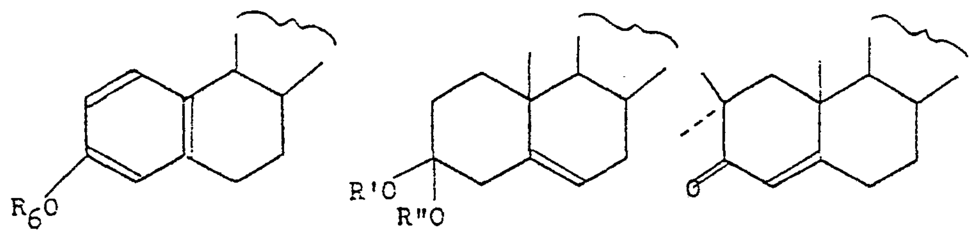

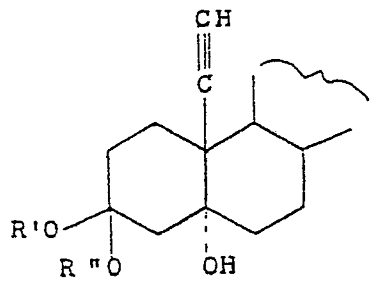

dans lequel R' et R" identiques ou différents représentent un radical méthyle ou éthyle ou R' et R" forment ensemble un radical éthylène ou triméthylène, par un réactif d'introduction du radical acétoxyle, pour obtenir un produit de formule (III') :

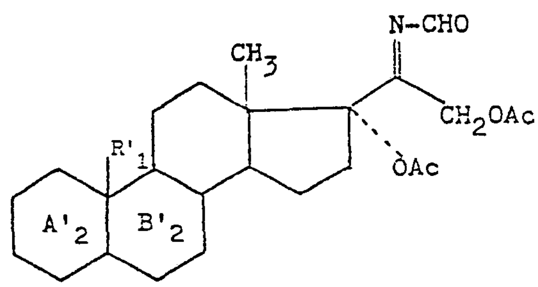

(III')

dans laquelle $R'_1$, Ac, $A'_2$ et $B'_2$ ont la signification précédente, produit que l'on traite par un agent d'hydrolyse, pour obtenir un produit de formule (IV') :

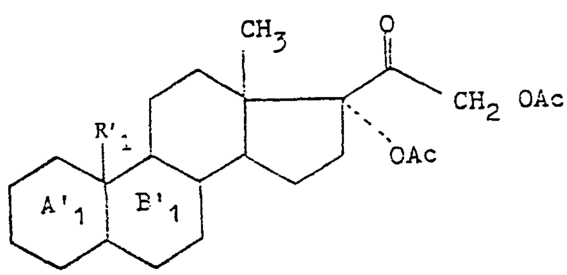

(IV')

dans laquelle $R'_1$, $A'_1$ et $B'_1$ ont la signification précédente, produit que l'on saponifie, pour obtenir un produit de formule (I') cherché.

Comme indiqué précédemment pour les produits de formule (II), les produits de formule (II') peuvent être sous la forme 20E, 20Z ou sous la forme d'un mélange 20E et 20Z.

Parmi les valeurs de $R_6$ on préfère les valeurs méthyle, éthyle ou benzyle et plus particulièrement méthyle. Parmi les valeurs de R' et R" on préfère la valeur éthylène.

Le réactif d'introduction du radical acétoxyle est le tétracétate de plomb ou l'iodosobenzène diacétate.

L'agent d'hydrolyse est de préférence l'acide chlorhydrique ou acétique.

La saponification des produits de formule (IV') est réalisée de préférence par action de la potasse suivie de l'action de l'acide acétique.

La présente invention a plus particulièrement pour objet, un procédé de préparation des produits de formule (I'') :

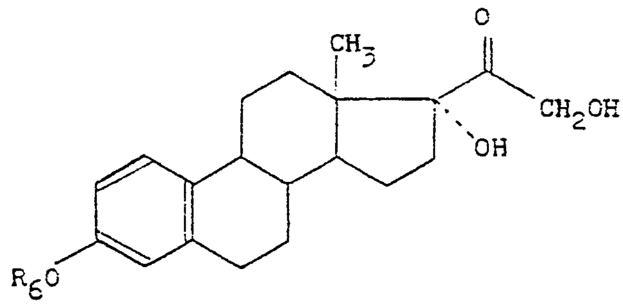

(I'')

dans laquelle $R_6$ a la signification indiquée ci-dessus, caractérisé en ce que l'on met en œuvre la procédé tel que décrit ci-dessus, au départ de produits de formule (II') dans laquelle les noyaux $A'_2$ et $B'_2$ sont les suivants :

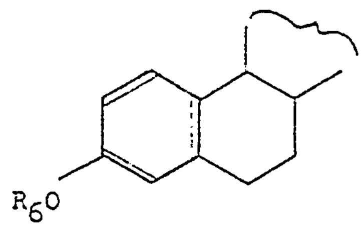

Parmi les différentes formes d'exécution du procédé ci-dessus, on préfère que le réactif d'introduction du radical acétoxyle soit le tétracétate de plomb.

Il apparaît que le procédé tel que décrit ci-dessus est à la fois d'une portée très générale puisqu'il vise la modification d'un substituant en position 17 du niveau stéroïde ce qui, en principe, n'affecte pas le reste des positions de la molécule, et d'autre part d'un grand intérêt puisqu'il permet d'accéder à la série des produits du type cortisonique 17α-hydroxy 17β-hydroxy acétyle. Comme on le verra par la suite, les produits de départ de formule (II) peuvent être aisément préparés à partir des produits 17-oxo selon un procédé non encore publié. Enfin le procédé de la présente demande est d'une mise en œuvre simple.

L'invention a également pour objet, au même titre de produits industriels nouveaux et notamment de produits nécessaires à la mise en œuvre du procédé ci-dessus les produits de formule ($IV'_A$) :

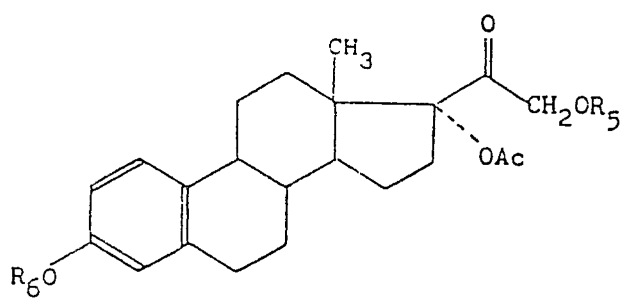

($IV'_A$)

dans laquelle $R_5$ et $R_6$ ont la signification indiquée précédemment.

Comme indiqué précédemment, les produits de formule (I) obtenus par le procédé sont pour la plupart des produits connus, du type cortisonique, dont les propriétés pharmacologiques ont été étudiées et permettent leur utilisation dans l'industrie pharmaceutique. Il en est par exemple ainsi de la 17α,21-dihydroxy pregn-4-ène 3,20-dione dite « substance S » obtenue dans la partie expérimentale.

Cependant certains produits de formule (I) sont nouveaux, il en est ainsi des produits de formule (I'') :

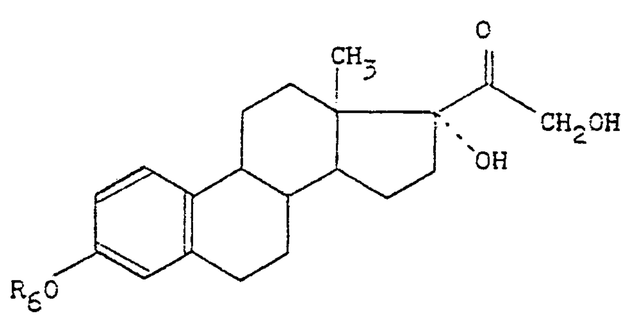

(I'')

dans laquelle $R_6$ représente un radical alkyle ayant de 1 à 8 atomes de carbone ou un radical aralkyle ayant de 7 à 12 atomes de carbone et plus spécialement de la 17α,21-dihydroxy 3-méthoxy 19-nor pregna 1,3,5(10) trien-20-one.

Les produits de formule (I") et en particulier la 17α,21-dihydroxy 3-méthoxy 19-nor pregna 1,3,5(10) trien-20-one peuvent être utilisés à titre de médicaments.

Ils peuvent également être utiles pour la préparation de produits pharmacologiquement actifs.

Les produits de formule (II) utilisés au départ du présent procédé peuvent être préparés comme suit :

a) On fait agir, sur un produit de formule (A)

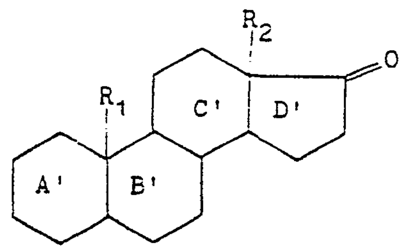

(A)

un produit de formule

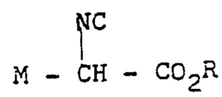

dans laquelle M représente un atome de métal alcalin et R représente un radical alkyle ayant de 1 à 18 atomes de carbone, pour obtenir un produit de formule (B)

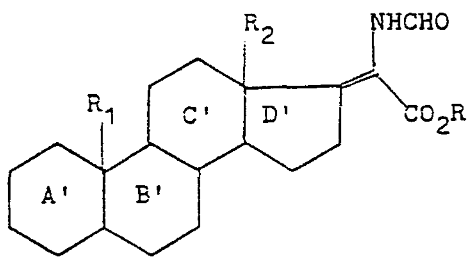

(B)

b) On traite le produit de formule (B) par un agent de réduction, de préférence de formule $M_1AlH_2(Oalc_1Oalc_2)_2$ dans laquelle $M_1$ représente un atome de métal alcalin et $alc_1$ et $alc_2$, identiques ou différents, représentent un radical alkyle ayant de 1 à 8 atomes de carbone, pour obtenir les produits de formule (II) dans laquelle $R_3$ représente un atome d'hydrogène.

Les produits de formule (II) dans laquelle $R_3$ représente un groupement protecteur du radical hydroxyle peuvent être obtenus par action, selon des méthodes connues, d'un dérivé réactif du groupement protecteur. On utilise de préférence le chlorure d'acétyle ou l'anhydride acétique.

Les exemples suivants illustrent l'invention sans toutefois la limiter :

Exemple 1

17α,21-dihydroxy pregn-4-ène 3,20-dione.

Stade A : (20Z) 21-acétoxy 3,3-éthylène dioxy 20-formylamino pregna 5,17 (20)-diène.

On met en suspension 3,54 g de (20Z) 3,3-éthylène dioxy 20-formyl amino pregna 5,17(20)-dièn-21-ol dans 35 $cm^3$ de pyridine et ajoute 17 $cm^3$ d'anhydride acétique. On agite la suspension à température ambiante. Après une heure trente, on détruit l'excès d'anhydride acétique par addition de quelques centimètres cubes d'eau, puis dilue à l'eau jusqu'à 250 $cm^3$, on essore, lave le précipité à l'eau, sèche et obtient 3,8 g de cristaux fondant à 216 °C. Après recristallisation dans le mélange chlorure de méthylène-éthanol, on obtient un échantillon analytique F = 220 °C.

Analyse : $C_{26}H_{39}O_7N$

Calculé : C % 70,40 H % 8,40 N % 3,15

Trouvé : C % 70,2 H % 8,4 N % 3,2

Stade B : 17α,21-diacétoxy 3,3-éthylène dioxy 20-formylimino pregn-5-ène.

On met en suspension 3,57 g de composé obtenu au stade A dans 200 $cm^3$ de benzène anhydre et ajoute en une seule fois 4 g de tétracétate de plomb sec. On agite le mélange pendant 50 minutes, verse dans une solution de carbonate acide de sodium puis émulsionne les deux phases. On essore et décante la phase aqueuse que l'on réextrait à l'acétate d'éthyle. On réunit les phases organiques, lave à l'eau, sèche, évapore le solvant et obtient 4 g de produit que l'on dissout dans du dichlorométhane à 1 % de triéthylamine. On dilue la solution à l'éther isopropylique et concentre pour chasser le dichlorométhane. On obtient une suspension que l'on glace et essore. On recueille 3,24 g de produit. F = 192 °C.

Analyse : $C_{28}H_{39}O_7N$

| | | | |
|---|---|---|---|
| Calculé : | C % 67,04 | H % 7,83 | N % 2,79 |
| Trouvé : | C % 67,1 | H % 7,8 | N % 2,7 |

Stade C : 17α,21-dihydroxy prégn-4-ène 3,20-dione.

On met en suspension 250 mg de produit obtenu au stade B dans 2,5 $cm^3$ d'acide acétique et 1 $cm^3$ d'eau. On agite à température ambiante et après 45 minutes dilue à l'eau, verse dans une solution de carbonate acide de sodium et extrait au dichlorométhane. Après séchage et évaporation du solvant on obtient 250 mg de produit brut. Ce produit est repris dans 5 $cm^3$ de tétrahydrofuranne, on ajoute 1 $cm^3$ d'acide chlorhydrique 2N, porte à 70 °C pendant 20 minutes, puis glace, dilue à l'eau, extrait au dichlorométhane, lave la phase organique, sèche, évapore à sec et obtient 220 mg de 17α,21-diacétoxy pregn-4-èn 3,20-dione.

Le produit est repris dans 6 $cm^3$ de méthanol, on refroidit la suspension au bain de glace et ajoute 0,2 $cm^3$ de potasse méthanolique (à 15 g pour 100 $cm^3$). Après 90 minutes d'agitation, on ajoute 0,2 $cm^3$ d'acide acétique, dilue à l'eau, extrait au dichlorométhane, lave à l'eau, sèche, évapore le solvant et obtient 180 mg de produit attendu. Après purification sur silice (éluant benzène-acétate d'éthyle 6-4) on obtient 130 mg de produit attendu F = 218 °C.

Le (20Z) 3,3-éthylène dioxy 20-formylamino pregna 5,17$^{(20)}$-dien-21-ol utilisé au départ de l'exemple 1 a été obtenu comme suit :

a) (20Z) 3,3-éthylène dioxy 20-formylamino pregna-5,17$^{(20}$U2)-diène-21-oate d'éthyle.

On agite dans un bain d'eau à 10 °C, un mélange de 2,5 $cm^3$ d'isocyanate d'éthyle conservé sous atmosphère inerte et 40 $cm^3$ de tétrahydrofuranne anhydre, ajoute goutte à goutte 8,6 $cm^3$ d'une solution 2,6 M de ter-butylate de potassium dans le tétrahydrofuranne et rince avec 5 $cm^3$ de tétrahydrofuranne anhydre. On obtient une suspension qui est agitée 10 minutes à + 15 °C. On ajoute ensuite, en une seule fois, 5,8 g de 3,3-éthylène dioxy androst 5-èn-17-one, puis agite à la température ambiante. On laisse une heure puis verse le mélange réactionnel dans une solution saturée de chlorure d'ammonium et extrait au dichlorométhane. On lave à l'eau, sèche, évapore sous pression réduite et obtient 8,6 g de produit brut. On empâte au reflux dans 50 $cm^3$ d'acide d'éthyle pendant 10 minutes, glace, essore, lave à l'acétate d'éthyle et obtient 4,55 g de composé attendu qui, après recristallisation dans un mélange dichlorométhane-acétate d'éthyle, donne 4,05 g de produit pur F = 214 °C.

On réunit les liqueurs mères de cristallisation et évapore à sec. On chromatographie le résidu sur silice (éluant-benzène-acétate 7-3). On recueille encore 3,3 g du produit attendu que l'on empâte dans l'acétate d'éthyle pour obtenir 2,62 g de produit pur. On sépare encore 0,15 g de produit à partir des liqueurs mères. On obtient ainsi au total 6,82 g de produit attendu.

b) (20Z) 3,3-éthylène dioxy 20-formylamino pregna 5,17$^{(20)}$-dien 21-ol.

On dissout sous azote 2,26 g de (20Z) 3,3-éthylène dioxy 20-formylamino pregna 5,17(20)-dièn 21-oate d'éthyle obtenu précédemment dans 33 $cm^3$ de tétrahydrofuranne anhydre. On refroidit la solution à + 3 °C et, sous agitation, ajoute goutte à goutte en 20 minutes 3,5 $cm^3$ de solution toluènique environ 3,5 M de dihydro bis (2-méthoxyéthoxy) aluminate de sodium.

On agite le mélange à 3 °C sous azote pendant deux heures puis ajoute lentement 22 $cm^3$ d'éthanol puis 1 g de borohydrure de sodium. On agite à température ambiante pendant 45 minutes puis ajoute une solution de sel de Seignette (tartrate double de potassium et de sodium) et extrait avec du dichlorométhane. Après rinçage et séchage, on évapore à sec et obtient 2,08 g de produit brut attendu que l'on recristallise dans un mélange dichlorométhane-éther isopropylique pour obtenir 1,78 g de produit purifié.

Après nouvelle recristallisation dans l'éthanol on obtient un échantillon analytique F = 260 °C.

## Exemple 2

17α,21-dihydroxy 3-méthoxy 19-nor pregna 1,3,5$^{(10)}$-trien-20-one.

Stade A : (20Z) 21-acétoxy 20-formamido 3-méthoxy 19-nor pregna 1,3,5(10),17(20)-tétraène.

On met en suspension 5,8 g de (20Z) 20-formamido 3-méthoxy 19-nor pregna 1,3,5(10),17(20)-tétraen-21-ol dans 12 $cm^3$ de pyridine et 12 $cm^3$ d'anhydride acétique. On agite à température ambiante. Après 4 heures la solution est diluée progressivement à l'eau jusqu'à cristallisation. On essore, lave à l'eau, sèche sous pression réduite et obtient 6,65 g de cristaux.

On recristallise le produit dans l'éthanol et obtient le produit attendu. Un échantillon analytique est obtenu par recristallisation dans l'acétate d'éthyle. F = 125 °C.

Analyse : $C_{24}H_{31}NO_4$
Calculé : C % 72,52 H % 7,86 N % 3,52
Trouvé : C % 72,6 H % 7,9 N % 3,5

Le produit de départ du stade A a été obtenu comme suit :

On dissout 1,6 g de (20Z) 20-formamido 3-méthoxy 19-nor pregna 1,3,5(10)17(20)-tétraèn-21-oate d'éthyle, préparé selon le procédé indiqué par U. Schollkopf et K. Hantke chem. Ber., 109, 3964 (1976), dans 32 $cm^3$ de tétrahydrofuranne anhydre. La solution est agitée à 0 °C pendant deux heures avec 200 mg de borohydrure de potassium et 200 mg d'hydrure double d'aluminium et de lithium. On ajoute ensuite de l'éthanol, goutte à goutte, pour décomposer l'excès d'hydrure double d'aluminium et de lithium, on agite encore une heure à la température ambiante, puis dilue le mélange réactionnel avec une solution de sel de Seignette (tartrate double de potassium et de sodium) et extrait avec de l'acétate d'éthyle. On filtre les extraits, les lave à l'eau, les sèche et les distille à sec. On triture le résidu obtenu avec de l'éther isopropylique, glace la suspension, essore, lave à l'éther isopropylique et sèche.

On obtient 1,28 g du produit recherché que l'on peut utiliser tel que
l'échantillon analytique a été obtenu par recristallisation dans du méthanol, F = 191 °C.

Stade B : 17α,21-diacétoxy 20-formylimino 3-méthoxy 19-nor pregna 1,3,5 (10) triène.

On dissout 400 mg de produit obtenu au stade A dans 50 $cm^3$ de benzène anhydre sous agitation. On ajoute 665 mg de tétracétate de plomb sec. Après 30 minutes de réaction à température ambiante, on ajoute de l'eau et filtre le mélange réactionnel. On recueille le filtrat, décante la phase aqueuse et lave avec une solution de carbonate acide de sodium.

On sèche la phase organique, filtre et distille à sec. Par purification sur silice (éluant benzène-acétate d'éthyle 9-1) puis recristallisation dans l'éther isopropylique, on obtient 300 mg de produit attendu. Un échantillon analytique a été obtenu par recristallisation dans un mélange de dichlorométhane et d'éther isopropylique puis dans le méthanol. F = 135 °C.

Analyse : $C_{26}H_{33}O_6N$
Calculé : C % 68,55 H % 7,30 N % 3,07
Trouvé : C % 68,4 H % 7,3 N % 3,0

Stade C : 17α,21-diacétoxy 3-méthoxy 19-nor pregna 1,3,5 (10) trien-20-one.

On dissout 120 mg de produit obtenu au stade B dans 2 $cm^3$ d'acide acétique et 1 $cm^3$ d'eau. On chauffe 3 heures au bain-marie à 100 °C. On refroidit, dilue à l'eau et extrait au dichlorométhane. On lave la phase organique à l'eau, sèche, évapore à sec et obtient 100 mg de produit attendu. Ce produit est purifié sur silice (éluant cyclohexane-acétate d'éthyle 7-3). On sépare 90 mg de produit purifié qui, après cristallisation dans le méthanol donne 55 mg de produit pur F = 162 °C.

Stade D : 17α,21-dihydroxy 3-méthoxy 19-nor pregna 1,3,5 (10) trien-20-one.

On fait barboter de l'azote dans une suspension de 980 mg de produit obtenu comme au stade C ci-dessus dans 7 $cm^3$ de méthanol. Après 15 minutes on refroidit la suspension par un bain de glace et ajoute 1 $cm^3$ de potasse méthanolique à 15 g pour 100 $cm^3$. On agite le mélange réactionnel sous azote au bain de glace pendant une heure. On ajoute ensuite 0,2 $cm^3$ d'acide acétique, dilue à l'eau et extrait avec du dichlorométhane. On lave à l'eau la phase organique, sèche et évapore sous pression réduite. On obtient 840 mg de produit brut.

Le produit est chromatographié sur silice en éluant avec un mélange benzène-acétate d'éthyle 7-3. On obtient 535 mg de produit cristallisé. On obtient un échantillon analytique par recristallisation dans l'éthanol aqueux puis dans le dichlorométhane, F = 177 °C.

Analyse : $C_{21}H_{28}O_4$
Calculé : C % 73,22 H % 8,19
Trouvé : C % 73,1 H % 8,4

**Revendications** (pour les Etats contractants : CH, DE, FR, GB, IT, LI, NL, SE)

1. Procédé de préparation de produits de formule (I)

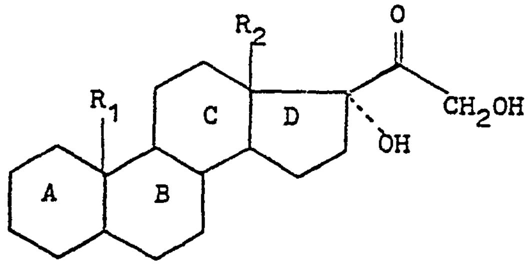

(I)

dans laquelle $R_1$ représente un atome d'hydrogène, un radical alkyle renfermant de 1 à 4 atomes de carbone substitué éventuellement par une fonction oxygénée ou azotée ou par un atome d'halogène ou $R_1$ représente un radical alkényle ou alkynyle renfermant de 2 à 4 atomes de carbone, $R_2$ représente un radical alkyle renfermant de 1 à 4 atomes de carbone, les noyaux A, B, C, D portent éventuellement une ou plusieurs doubles liaisons et sont éventuellement substitués par une ou plusieurs fonctions hydroxyle ou cétone, par un ou plusieurs atomes d'halogène, par un ou plusieurs radicaux alkyle ou alkyloxy renfermant de 1 à 4 atomes de carbone ou par un ou plusieurs radicaux alkényle ou alkynyle renfermant de 2 à 4 atomes de carbone, caractérisé en ce que l'on traite un produit de formule (II)

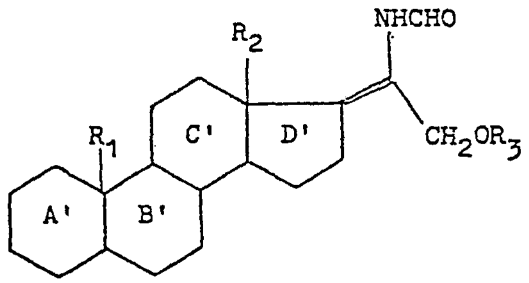

(II)

dans laquelle $R_1$ et $R_2$ ont la signification précédente et les noyaux A', B', C' et D' ont les mêmes valeurs que précédemment décrite pour A, B, C, D avec la possibilité supplémentaire d'être substitués par des fonctions hydroxyle ou cétone protégées et $R_3$ représente un atome d'hydrogène ou $R'_3$, $R'_3$ représentant un groupement protecteur du radical hydroxyle, par un réactif d'introduction du radical acétoxyle, pour obtenir un produit de formule (III) :

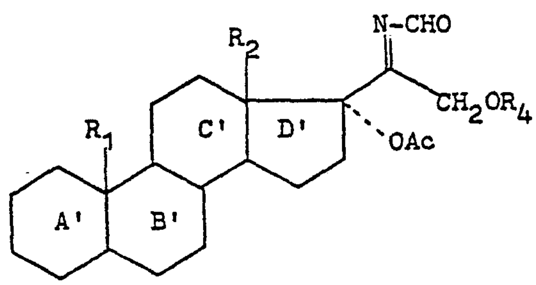

(III)

dans laquelle $R_4$ représente $R'_3$ ou un radical acétyle et Ac représente un radical acétyle, produit que l'on traite par un agent d'hydrolyse, pour obtenir un produit de formule (IV) :

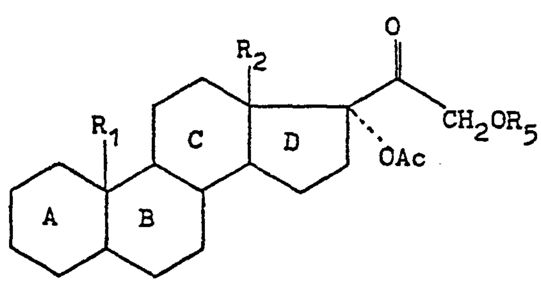

(IV)

dans laquelle $R_1$, $R_2$, Ac, A, B, C et D ont la signification précédente et $R_5$ représente un atome d'hydrogène, un groupement protecteur du radical hydroxyle non facilement hydrolysable ou un radical acétyle, produit que l'on traite de manière à éliminer le radical acétyle en 17α et le groupement $R_5$ lorsque celui-ci ne représente pas un atome d'hydrogène, pour obtenir un produit de formule (I).

2. Procédé selon la revendication 1, caractérisé en ce que l'on met en œuvre le procédé au départ de produits de formule (II) dans laquelle $R_2$ représente un radical méthyle et $R_1$ représente un atome d'hydrogène ou un radical méthyle ou éthynyle.

3. Procédé selon les revendications 1 ou 2 de préparation des produits de formule (I') :

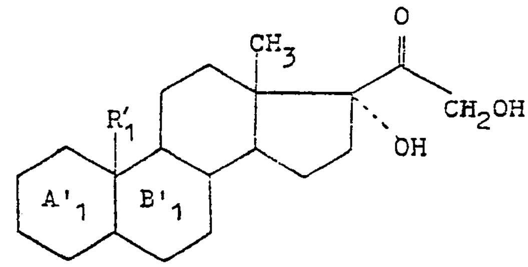

(I')

dans laquelle $R'_1$, $A'_1$ et $B'_1$ sont tels que les noyaux $A'_1$ et $B'_1$ sont choisis dans le groupe formé par les systèmes suivants :

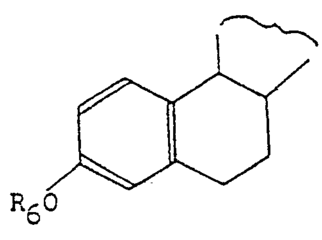
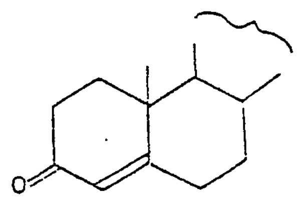
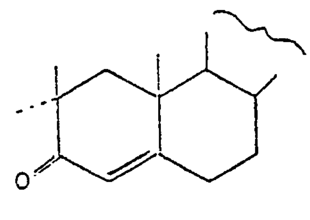
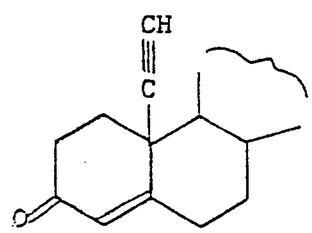

dans lequel $R_6$ représente un radical alkyle ayant de 1 à 8 atomes de carbone ou un radical aralkyle ayant de 7 à 12 atomes de carbone, caractérisé en ce que l'on traite un produit de formule (II')

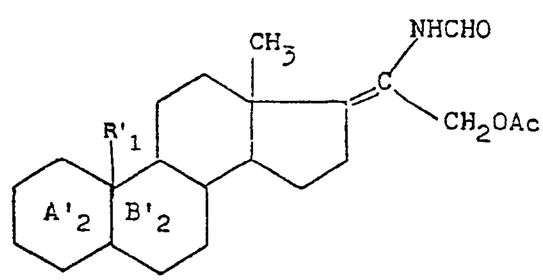

(II')

dans laquelle $R'_1$ a la définition indiquée précédemment, Ac représente un radical acétyle et les noyaux $A'_2$ et $B'_2$ sont choisis dans le groupe formé par les systèmes suivants :

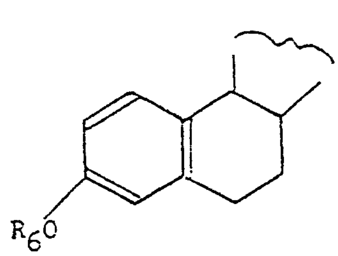
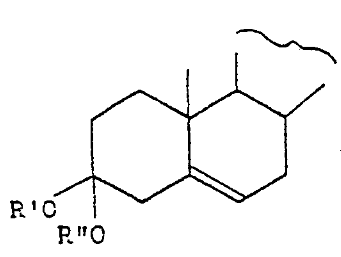
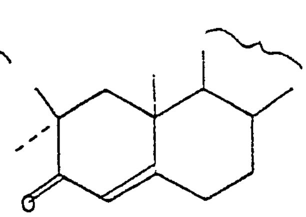

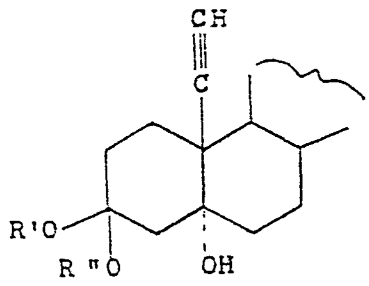

dans lequel R' et R'', identiques ou différents, représentent un radical méthyle ou éthyle ou R' et R'' forment ensemble un radical éthylène ou triméthylène, par un réactif d'introduction du radical acétoxyle, pour obtenir un produit de formule (III') :

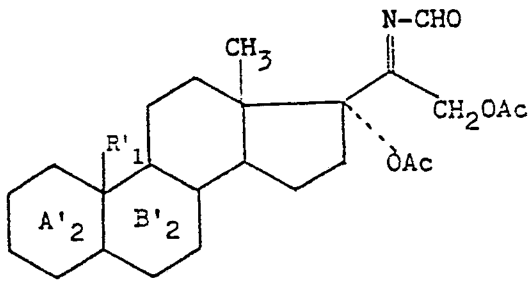

(III')

dans laquelle $R'_1$, Ac, $A'_2$ et $B'_2$ ont la signification précédente, produit que l'on traite par un agent d'hydrolyse, pour obtenir un produit de formule (IV') :

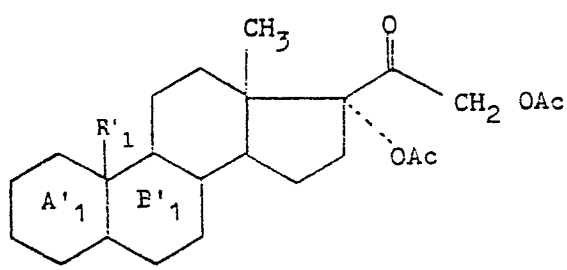

(IV')

dans laquelle $R'_1$, $A'_1$ et $B'_1$ ont la signification précédente, produit que l'on saponifie, pour obtenir un produit de formule (I') cherché.

4. Procédé selon les revendications 1 à 3 de préparation des produits de formule (I'')

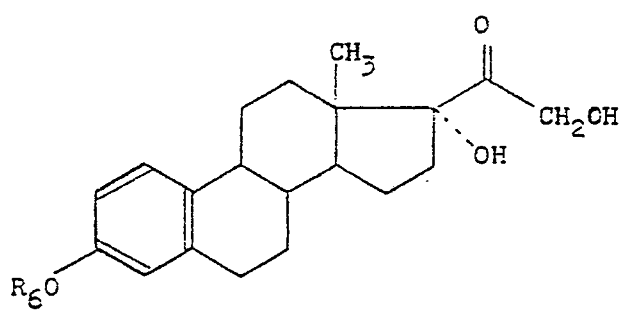

(I'')

dans laquelle $R_6$ a la signification indiquée à la revendication 3, caractérisé en ce que l'on met en œuvre le procédé tel que décrit aux revendications 1 à 3, au départ de produits de formule (II') dans laquelle les noyaux $A'_2$ et $B'_2$ sont les suivants :

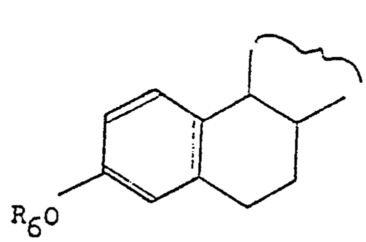

5. Procédé selon l'une quelconque des revendications 1 à 4, caractérisé en ce que le réactif d'introduction du radical acétoxyle est le tétracétate de plomb.

6. Procédé selon la revendication 3 ou 4, caractérisé en ce que le radical $R_6$ représente un radical méthyle et les radicaux R' et R" forment ensemble un radical éthylène.

7. A titre de produits industriels nouveaux, les produits de formule ($IV'_A$) :

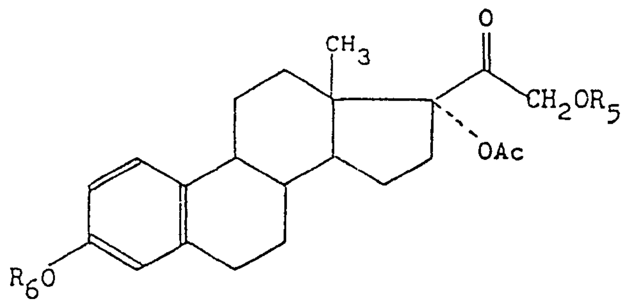

($IV'_A$)

dans laquelle $R_5$ et $R_6$ ont les significations indiquées aux revendications 1 et 3.

**Revendications** (pour l'Etat contractant AT)

1. Procédé de préparation de produits de formule (I)

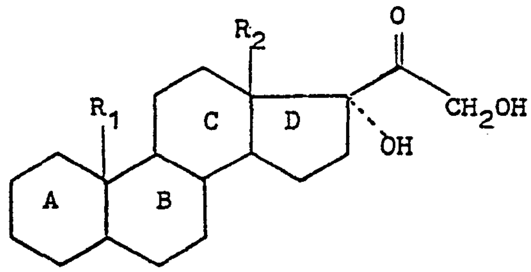

(I)

dans laquelle $R_1$ représente un atome d'hydrogène, un radical alkyle renfermant de 1 à 4 atomes de carbone substitué éventuellement par une fonction oxygénée ou azotée ou un atome d'halogène ou $R_1$ représente un radical alkényle ou alkynyle renfermant de 2 à 4 atomes de carbone, $R_2$ représente un radical alkyle renfermant de 1 à 4 atomes de carbone, les noyaux A, B, C, D portent éventuellement une ou plusieurs doubles liaisons et sont éventuellement substitués par une ou plusieurs fonctions hydroxyle ou cétone, par un ou plusieurs atomes d'halogène, par un ou plusieurs radicaux alkyle ou alkyloxy renfermant de 1 à 4 atomes de carbone ou par un ou plusieurs radicaux alkényle ou alkynyle renfermant de 2 à 4 atomes de carbone, caractérisé en ce que l'on traite un produit de formule (II)

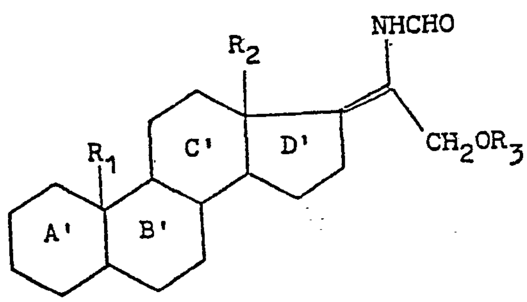

(II)

dans laquelle $R_1$ et $R_2$ ont la signification précédente et les noyaux A', B', C' et D' ont les mêmes valeurs que précédemment décrite pour A, B, C, D avec la possibilité supplémentaire d'être substitués par des fonctions hydroxyle ou cétone protégées et $R_3$ représente un atome d'hydrogène ou $R'_3$, $R'_3$ représentant un groupement protecteur du radical hydroxyle, par un réactif d'introduction du radical acétoxyle, pour obtenir un produit de formule (III) :

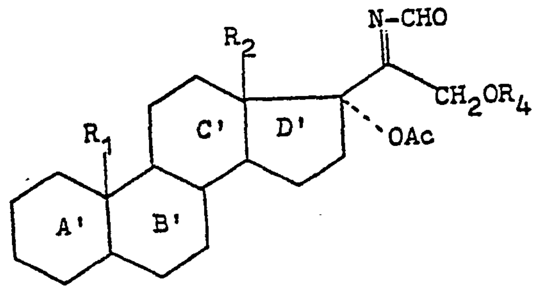

(III)

dans laquelle $R_4$ représente $R'_3$ ou un radical acétyle et Ac représente un radical acétyle, produit que l'on traite par un agent d'hydrolyse, pour obtenir un produit de formule (IV) :

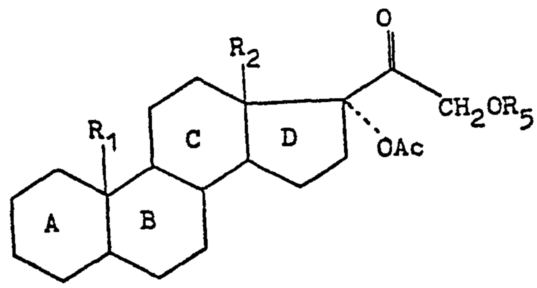

(IV)

dans laquelle $R_1$, $R_2$, Ac, A, B, C et D ont la signification précédente et $R_5$ représente un atome d'hydrogène, un groupement protecteur du radical hydroxyle non facilement hydrolysable ou un radical acétyle, produit que l'on traite de manière à éliminer le radical acétyle en 17α et le groupement $R_5$ lorsque celui-ci ne représente pas un atome d'hydrogène, pour obtenir un produit de formule (I).

2. Procédé selon la revendication 1, caractérisé en ce que l'on met en œuvre le procédé au départ de produits de formule (II) dans laquelle $R_2$ représente un radical méthyle et $R_1$ représente un atome d'hydrogène ou un radical méthyle ou éthynyle.

3. Procédé selon les revendications 1 ou 2 de préparation des produits de formule (I') :

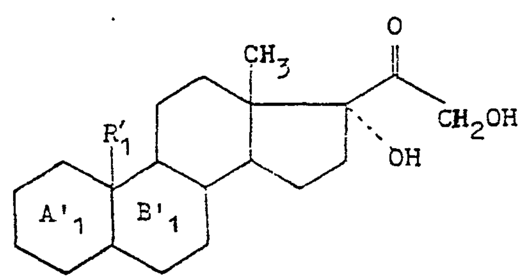

(I')

dans laquelle $R'_1$, $A'_1$ et $B'_1$ sont tels que les noyaux $A'_1$ et $B'_1$ sont choisis dans le groupe formé par les systèmes suivants :

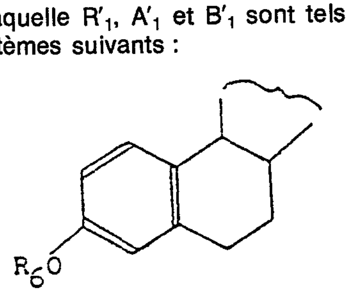

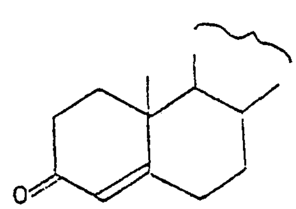

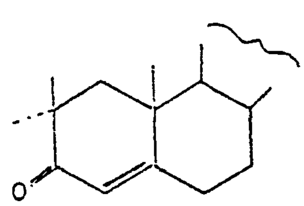

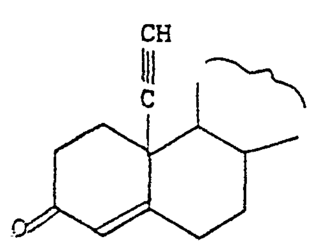

dans lequel $R_6$ représente un radical alkyle ayant de 1 à 8 atomes de carbone ou un radical aralkyle ayant de 7 à 12 atomes de carbone, caractérisé en ce que l'on traite un produit de formule (II')

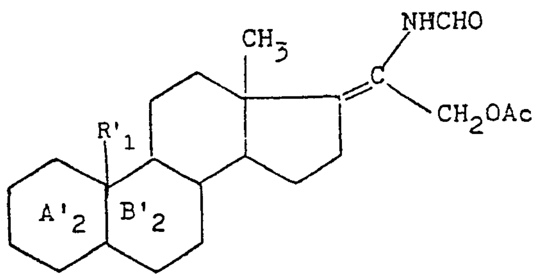

(II')

dans laquelle $R'_1$ a la définition indiquée précédemment, Ac représente un radical acétyle et les noyaux $A'_2$ et $B'_2$ sont choisis dans le groupe formé par les systèmes suivants :

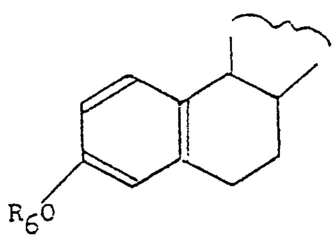

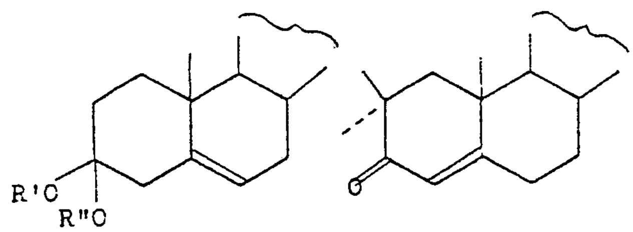

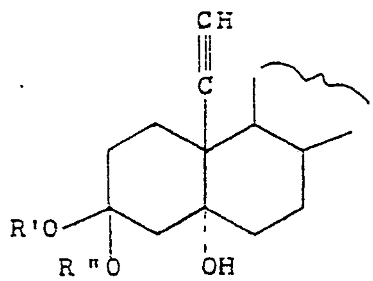

dans lequel R' et R'', identiques ou différents, représentent un radical méthyle ou éthyle ou R' et R'' forment ensemble un radical éthylène ou triméthylène, par un réactif d'introduction du radical acétoxyle, pour obtenir un produit de formule (III') :

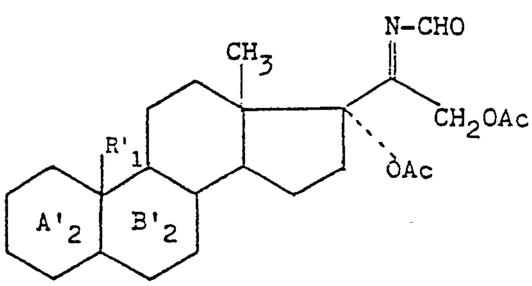

(III')

dans laquelle $R'_1$, Ac, $A'_2$ et $B'_2$ ont la signification précédente, produit que l'on traite par un agent d'hydrolyse, pour obtenir un produit de formule (IV') :

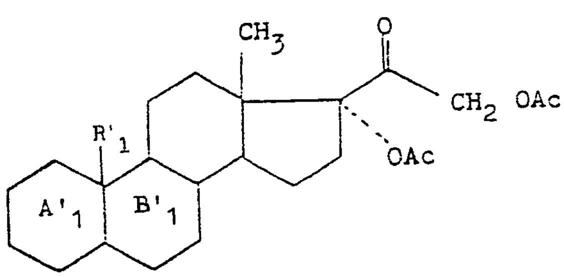

(IV')

dans laquelle $R'_1$, $A'_1$ et $B'_1$ ont la signification précédente, produit que l'on saponifie, pour obtenir un produit de formule (I') cherché.

4. Procédé selon les revendications 1 à 3 de préparation des produits de formule (I")

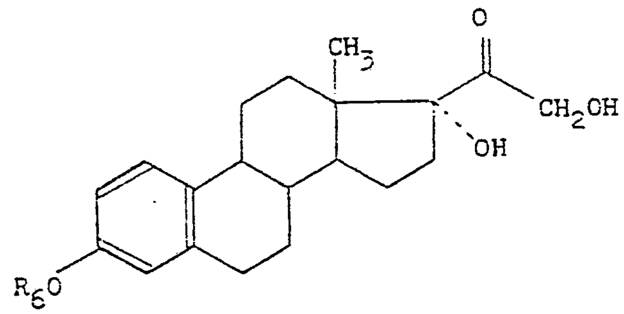

(I")

dans laquelle $R_6$ a la signification indiquée à la revendication 3, caractérisé en ce que l'on met en œuvre le procédé tel que décrit aux revendications 1 à 3, au départ de produits de formule (II') dans laquelle les noyaux $A'_2$ et $B'_2$ sont les suivants :

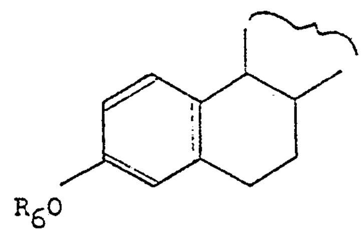

5. Procédé selon l'une quelconque des revendications 1 à 4, caractérisé en ce que le réactif d'introduction du radical acétoxyle est le tétracétate de plomb.

6. Procédé selon la revendication 3 ou 4, caractérisé en ce que le radical $R_6$ représente un radical méthyle et les radicaux R' et R" forment ensemble un radical éthylène.

**Claims** (for the Contracting States : CH, DE, FR, GB, IT, LI, NL, SE)

1. Preparation process for the products with the formula (I)

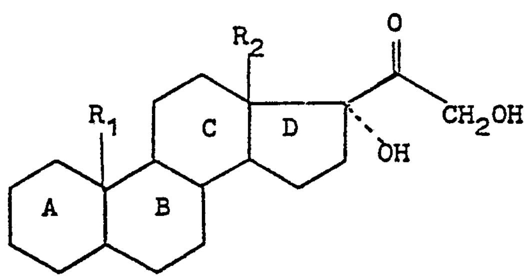

(I)

in which $R_1$ represents a hydrogen atom, an alkyl radical containing 1-4 carbon atoms, possibly substituted by an oxygenated or nitrogenated function, or by a halogen atom or $R_1$ represents an alkenyl or alkynyl radical containing 2-4 carbon atoms, $R_2$ represents an alkyl radical containing 1-4 carbon atoms, the nuclei A, B, C, D possibly carrying one or more double bonds and being possibly substituted by one or more hydroxyl or ketone functions, by one or more halogen atoms, by one or more alkyl or alkyloxy radicals containing 1-4 carbon atoms or by one or more alkenyl or alkynyl radicals containing 2-4 carbon atoms, characterized in that a product with the formula (II) :

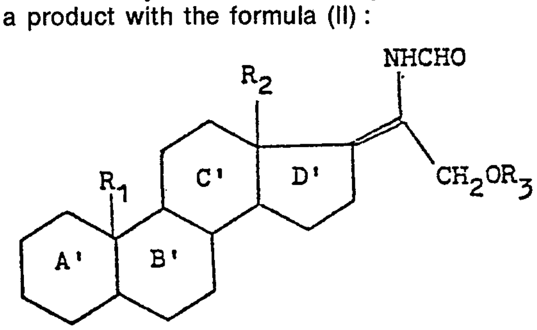

(II)

in which $R_1$ and $R_2$ have the same significance as previously and the nuclei A', B', C' and D' have the same values as previously desribed for A, B, C, D with the additional possibility of being substituted by protected hydroxyl or ketone functions, and $R_3$ represents a hydrogen atom or $R'_3$, $R'_3$ representing a protective group of the hydroxyl radical, is treated with a reagent for the introduction of the acetoxyl radical, so as to obtain a product with the formula (III) :

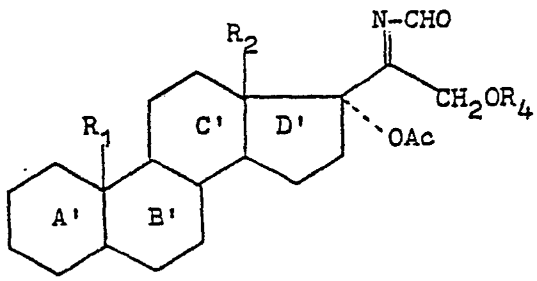

(III)

in which $R_4$ represents $R'_3$ or an acetyl radical and Ac represents an acetyl radical, which product is treated with a hydrolyzing agent so as to obtain a product with the formula (IV) :

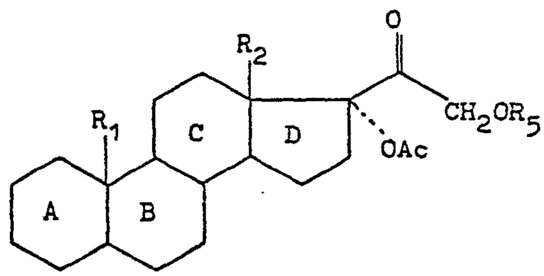

(IV)

in which $R_1$, $R_2$, Ac, A, B, C and D have the preceding significance and $R_5$ represents a hydrogen atom, a not-easily hydrolysable protective group of the hydroxyl radical, or an acetyl radical, which product is treated in such a way as to eliminate the acetyl radical in position 17α and the $R_5$ group when the latter does not represent a hydrogen atom, so as to obtain a product with the formula (I).

2. Process according to Claim 1, characterized in that the process is put into operation starting with the products with the formula (II) in which $R_2$ represents a methyl radical and $R_1$ represents a hydrogen atom or a methyl or ethynyl radical.

3. Process according to Claims 1 or 2 for preparation of products with the formula (I') :

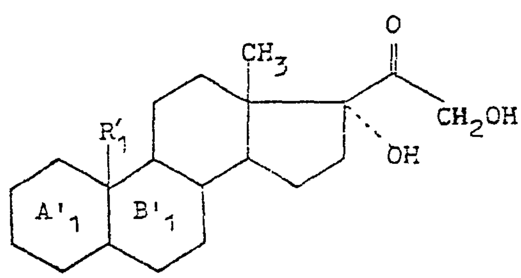

(I')

in which $R'_1$, $A'_1$ and $B'_1$ are such that the nuclei $A'_1$ and $B'_1$ are chosen from the group formed by the following systems :

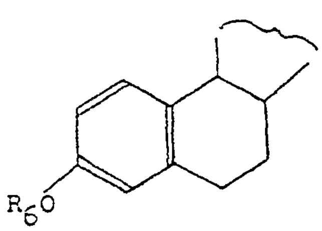

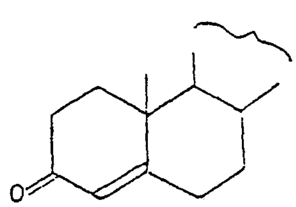

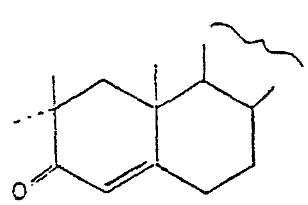

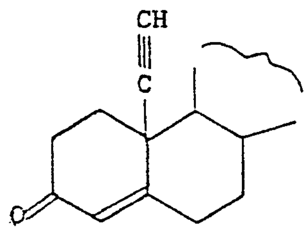

in which $R_6$ represents an alkyl radical containing 1-8 carbon atoms or an aralkyl radical containing 7-12 carbon atoms, characterized in that a product with the formula (II') :

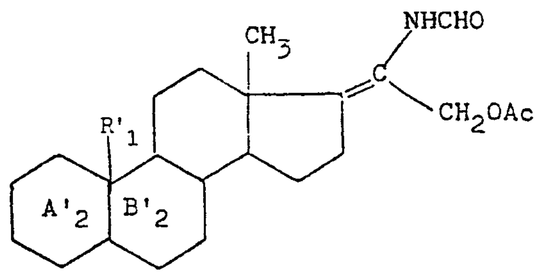

(II')

in which $R'_1$ has the definition previously indicated, Ac represents an acetyl radical and the nuclei $A'_2$ and $B'_2$ are chosen from the group formed by the following systems :

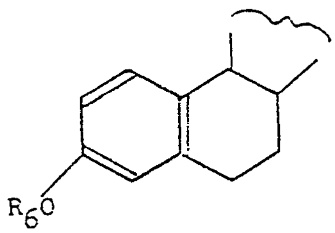

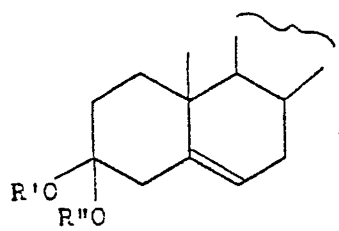

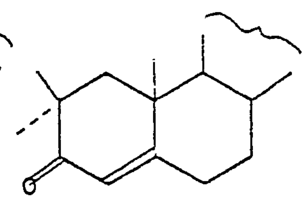

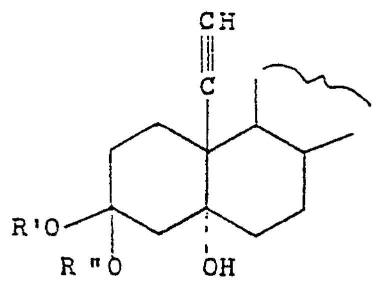

in which R' and R'', identical or different, each represent a methyl radical or an ethyl radical or R' and R'' together form an ethylene or trimethylene radical, is treated with a reagent for the introduction of the acetoxyl radical, so as to obtain a product with the formula (III') :

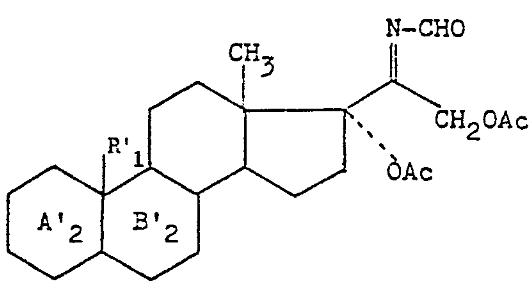

(III')

in which $R'_1$, Ac, $A'_2$ and $B'_2$ have the preceding significance, which product is treated with a hydrolyzing agent so as to obtain a product with the formula (IV') :

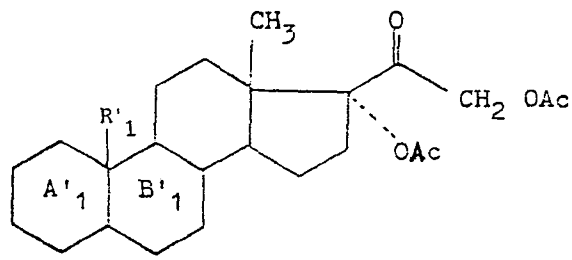

(IV')

in which $R'_1$, $A'_1$ and $B'_1$ have the preceding significance, which product is saponified so as to obtain a product with the formula (I') sought.

4. Process according to Claims 1-3 for preparation of products with the formula (I"):

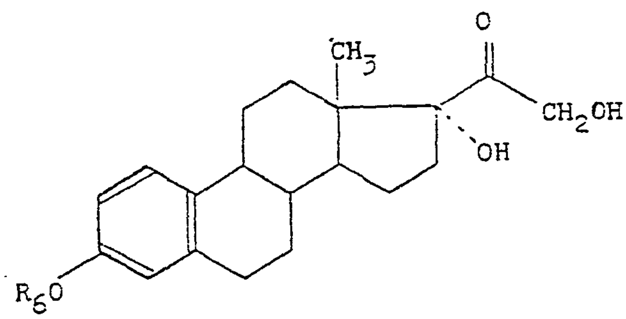

(I")

in which $R_6$ has the significance indicated in Claim 3, characterized in that the process is put into operation as described in Claims 1-3, starting with products with the formula (II') in which the nuclei $A'_2$ and $B'_2$ are the following:

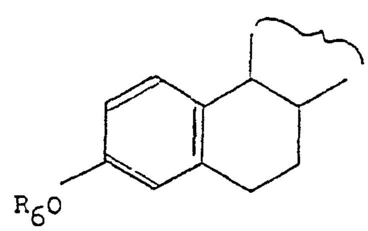

5. Process according to any one of Claims 1-4, characterized in that the reagent for the introduction of the acetoxyl radical is lead tetracetate.

6. Process according to Claim 3 or 4, characterized in that the radical $R_6$ represents a methyl radical and the radicals R' and R" together form an ethylene radical.

7. As new industrial products, the products with the formula ($IV'_A$):

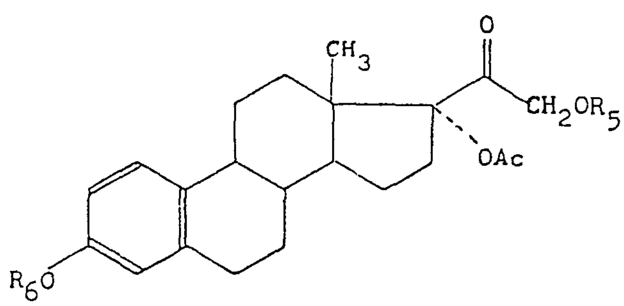

($IV'_A$)

in which $R_5$ and $R_6$ have the significances indicated in Claims 1 and 3.

**Claims** (for the Contracting State AT)

1. Preparation process for the products with the formula (I):

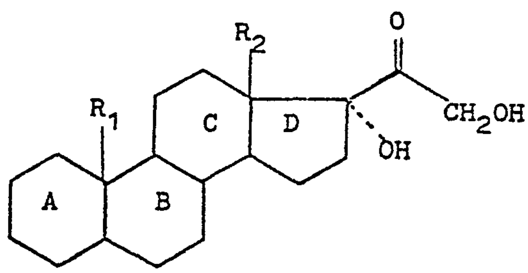

(I)

in which $R_1$ represents a hydrogen atom, an alkyl radical containing 1-4 carbon atoms possibly substituted by an oxygenated or nitrogenated function or by a halogen atom or $R_1$ represents an alkenyl or alkynyl radical containing 2-4 carbon atoms, $R_2$ represents an alkyl radical containing 1-4 carbon atoms, the nuclei A, B, C, D possibly carry one or more double bonds and are possibly substituted by one or more hydroxyl or ketone functions, by one or more halogen atoms, by one or more alkyl or alkyloxy radicals containing 1-4 carbon atoms or by one or more alkenyl or alkynyl radicals containing 2-4 carbon atoms, characterized in that a product with the formula (II)

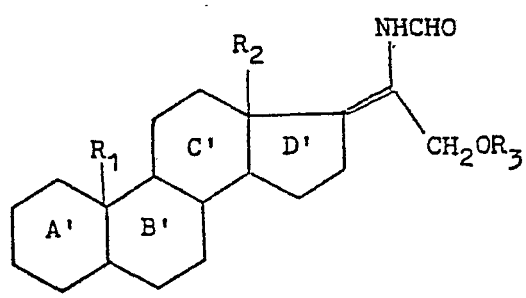

(II)

in which $R_1$ and $R_2$ have the preceding significance and the nuclei A', B', C' and D' have the same values as previously described for A, B, C, D with the additional possibility of being substituted by protectes hydroxyl or ketone functions and $R_3$ represents a hydrogen atom or $R'_3$, $R'_3$ representing a protective group of the hydroxyl radical, is treated with a reagent for the introduction of the acetoxyl radical, so as to obtain a product with the formula (III) :

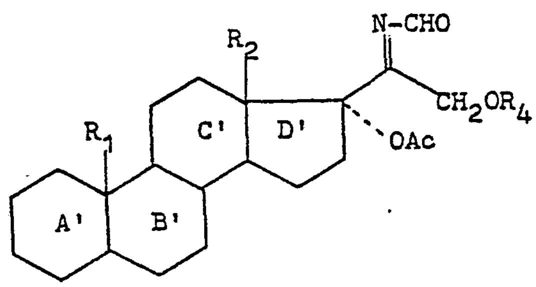

(III)

in which $R_4$ represents $R'_3$ or an acetyl radical and Ac represents an acetyl radical, which product is treated with a hydrolyzing agent so as to obtain a product with the formula (IV) :

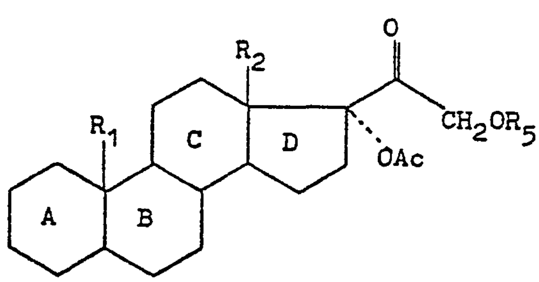

(IV)

in which $R_1$, $R_2$, Ac, A, B, C and D have the preceding significance and $R_5$ represents a hydrogen atom, a

not-easily hydrolysable protective group of the hydroxyl radical or an acetyl radical, which product is treated in such a way as to eliminate the acetyl radical in position 17α and the group $R_5$ when the latter does not represent a hydrogen atom, so as to obtain a product with the formula (I).

2. Process according to Claim 1, characterized in that the process is put into operation starting with products with the formula (II) in which $R_2$ represents a methyl radical and $R_1$ represents a hydrogen atom or a methyl or ethynyl radical.

3. Process according to Claims 1 or 2 for the preparation of products with the formula (I') :

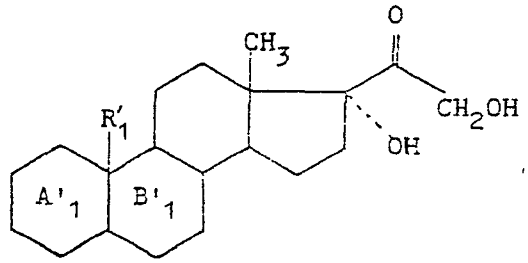

(I')

in which $R'_1$, $A'_1$ and $B'_1$ are such that the nuclei $A'_1$ and $B'_1$ are chosen from the group formed by the following systems :

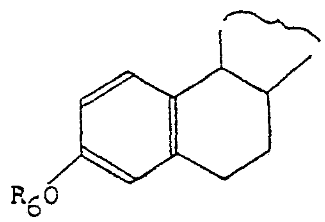

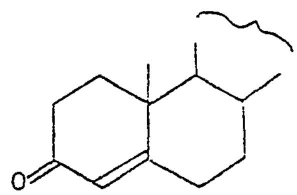

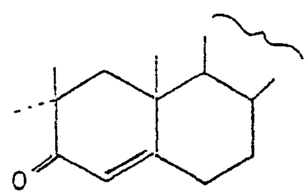

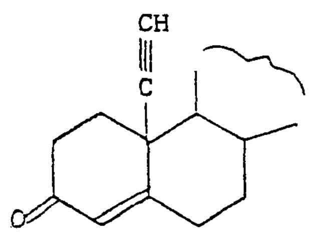

in which $R_6$ represents an alkyl radical containing 1-8 carbon atoms or an aralkyl radical containing 7-12 carbon atoms, characterized in that a product with the formula (II') :

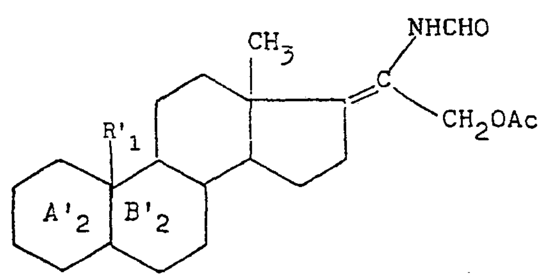

(II')

in which $R'_1$ has the definition indicated previously, Ac represents an acetyl radical and the nuclei $A'_2$ and $B'_2$ are chosen from the group formed by the following systems :

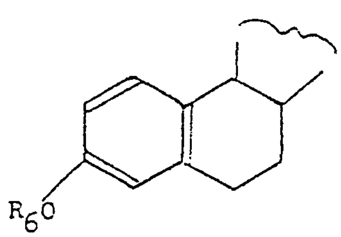

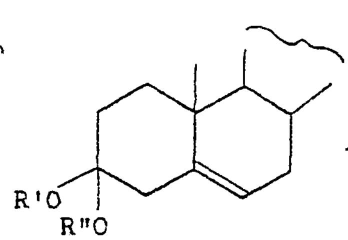

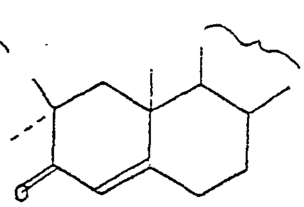

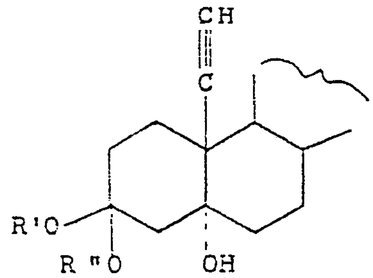

in which R′ and R″, identical or different, each represent a methyl or ethyl radical or R′ and R″ together form an ethylene or trimethylene radical, is treated with a reagent for the introduction of the acetoxyl radical, so as to obtain a product with the formula (III′) :

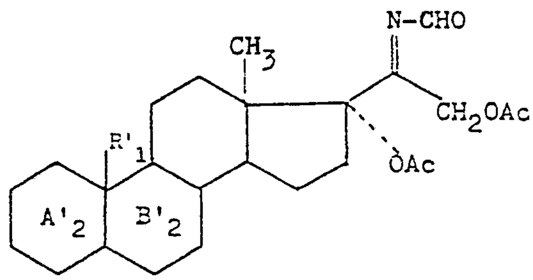

(III′)

in which $R'_1$, Ac, $A'_2$ and $B'_2$ have the preceding significance, which product is treated with a hydrolyzing agent so as to obtain a product with the formula (IV′) :

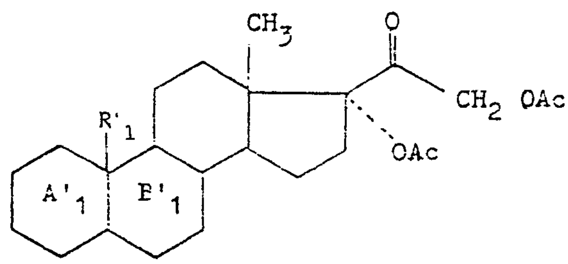

(IV′)

in which $R'_1$, $A'_1$ and $B'_1$ have the preceding significance, which product is saponified, so as to obtain a product with the formula (I′) sought.

4. Process according to Claims 1-3 for preparation of products with the formula (I″) :

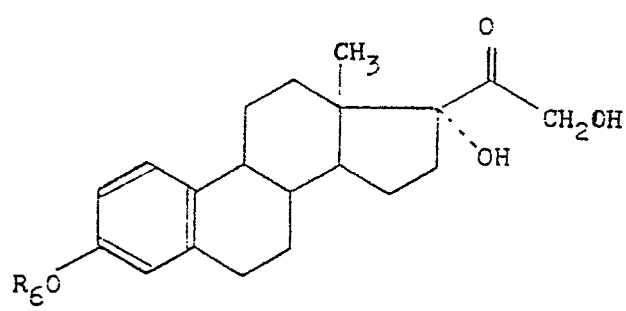

(I″)

in which $R_6$ has the significance indicated in Claim 3, characterized in that the process is put into operation as described in Claims 1-3, starting with products with the Formula (II′) in which the nuclei $A'_2$ and $B'_2$ are the following :

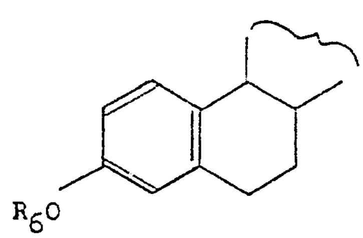

5. Process according to any one of Claims 1-4, characterized in that the reagent for the introduction of the acetoxyl radical is lead tetracetate.

6. Process according to Claim 3 or 4, characterized in that the radical $R_6$ represents a methyl radical and the radicals R' and R" together form an ethylene radical.

**Ansprüche** (für die Vertragsstaaten : CH, DE, FR, GB, IT, LI, NL, SE)

1. Verfahren zur Herstellung der Produkte der Formel (I)

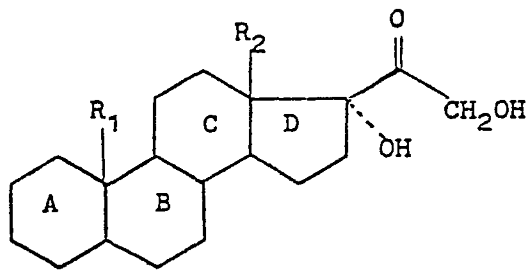

(I)

worin $R_1$ ein Wasserstoffatom, einen Alkylrest mit 1 bis 4 Kohlenstoffatomen, der gegebenenfalls durch eine Sauerstoff oder Stickstoff enthaltende Funktion oder durch ein Halogenatom substituiert ist, bedeutet oder $R_1$ einen Alkenyl- oder Alkinylrest mit 2 bis 4 Kohlenstoffatomen bedeutet, $R_2$ einen Alkylrest mit 1 bis 4 Kohlenstoffatomen bedeutet, die Ringe A, B, C und D gegebenenfalls eine oder mehrere Doppelbindungen enthalten und gegebenenfalls durch eine oder mehrere Hydroxyl- oder Ketonfunktionen, durch ein oder mehrere Halogenatome, durch einen oder mehrere Alkyl- oder Alkoxyreste mit 1 bis 4 Kohlenstoffatomen oder durch einen oder mehrere Alkenyl- oder Alkinylreste mit 2 bis 4 Kohlenstoffatomen substituiert sind, dadurch gekennzeichnet, daß man ein Produkt der Formel (II)

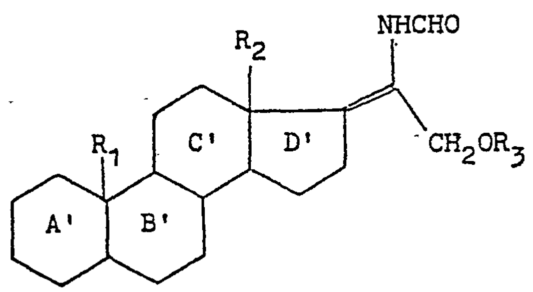

(II)

worin $R_1$ und $R_2$ die vorstehend angegebene Bedeutung besitzen und die Ringe A', B', C' und D' die vorstehend für A, B, C und D angegebenen Bedeutungen besitzen, mit der zusätzlichen Möglichkeit, durch geschützte Hydroxyl- oder Ketonfunktionen substituiert zu sein, und $R_3$ ein Wasserstoffatom oder $R'_3$ bedeutet, wobei $R'_3$ eine Schutzgruppe für den Hydroxylrest darstellt, mit einem Reagens zur Einführung eines Acetoxylrestes behandelt, um ein Produkt der Formel (III)

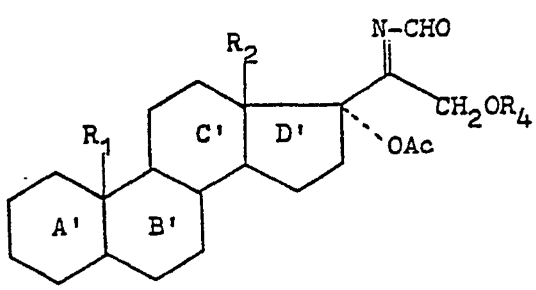

(III)

zu erhalten, worin $R_4$ für $R'_3$ oder einen Acetylrest steht und Ac einen Acetylerest bedeutet, welches Produkt man mit einem Hydrolysemittel behandelt, um ein Produkt der Formel (IV)

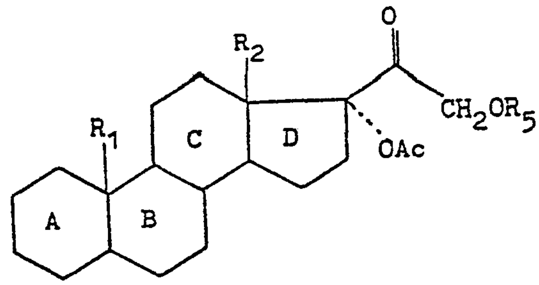

(IV)

zu erhalten, worin $R_1$, $R_2$, Ac, A, B, C und D die vorstehend angegebene Bedeutung besitzen und $R_5$ ein Wasserstoffatom, eine Schutzgruppe für den Hydroxylrest, die nicht leicht hydrolysierbar ist, oder einen Acetylrest bedeutet, welches Produkt man zur Entfernung des Acetylrestes in 17α-Stellung und der Gruppe $R_5$, wenn diese kein Wasserstoffatom bedeutet, behandelt, um ein Produkt der Formel (I) zu erhalten.

2. Verfahren gemäß Anspruch 1, dadurch gekennzeichnet, daß man von Produkten der Formel (II) ausgeht, worin $R_2$ einen Methylrest bedeutet und $R_1$ ein Wasserstoffatom oder einen Methyl- oder Ethinylrest darstellt.

3. Verfahren gemäß den Ansprüchen 1 oder 2 zur Herstellung der Produkte der Formel (I')

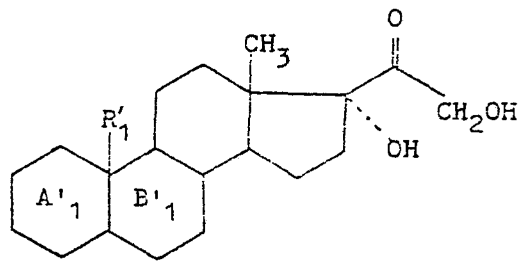

(I')

worin $R'_1$, $A'_1$ und $B'_1$ derart sind, daß die Ringe $A'_1$ und $B'_1$ ausgewählt sind aus der Gruppe der folgenden Systeme :

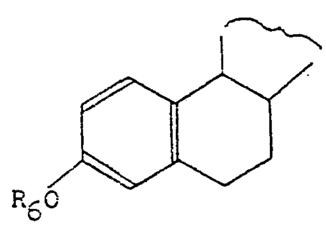

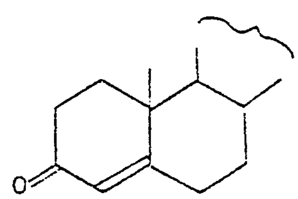

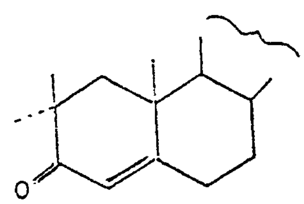

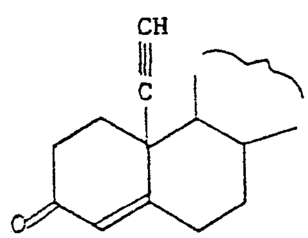

worin $R_6$ einen Alkylrest mit 1 bis 8 Kohlenstoffatomen oder einen Aralkylrest mit 7 bis 12 Kohlenstoffatomen bedeutet, dadurch gekennzeichnet, daß man ein Produkt der Formel (II')

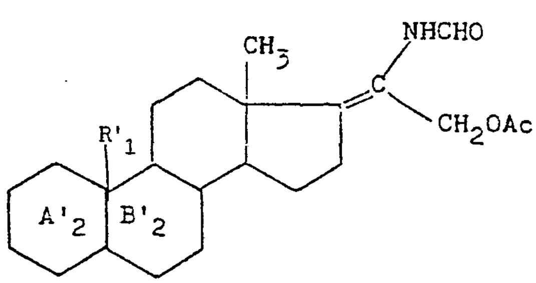

(II')

worin $R'_1$ die vorstehend angegebene Bedeutung besitzt, Ac einen Acetylrest bedeutet und die Ringe $A'_2$ und $B'_2$ ausgewählt sind aus der Gruppe der Folgenden Systeme :

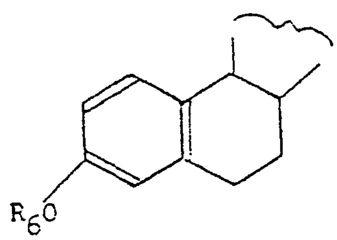

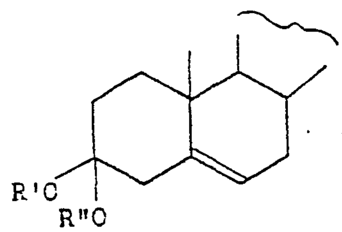

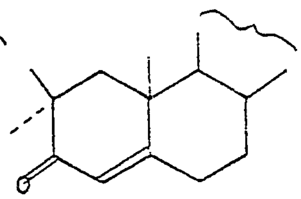

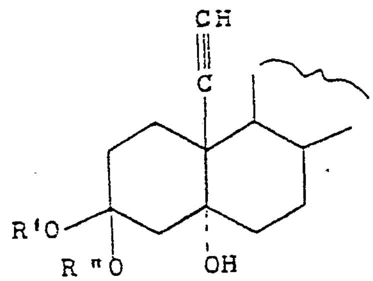

worin R' und R", die gleich oder verschieden sein können, einen Methyl- oder Ethylrest bedeuten oder R' und R" gemeinsam einen Ethylen- oder Trimethylenrest bilden, mit einem Reagens zur Einführung eines Acetoxylrestes behandelt, um ein Produkt der Formel (III')

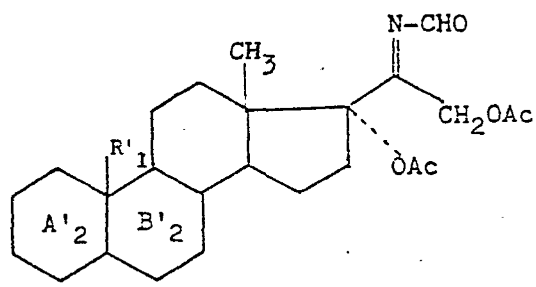

(III')

zu erhalten, worin $R'_1$, Ac, $A'_2$ und $B'_2$ die vorstehend angegebene Bedeutung besitzen, welches Produkt man mit einem Hydrolysemittel behandelt, um ein Produkt der Formel (IV')

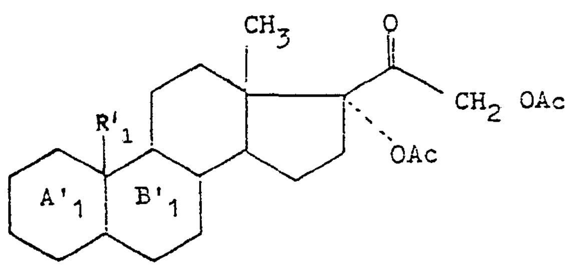

(IV')

zu erhalten, worin $R'_1$, $A'_1$ und $B'_1$ die vorstehend angegebene Bedeutung besitzen, welches Produkt man verseift, um ein gewünschtes Produkt der Formel (I') zu erhalten.

4. Verfahren gemäß den Ansprüchen 1 bis 3 zur Herstellung der Produkte der Formel (I")

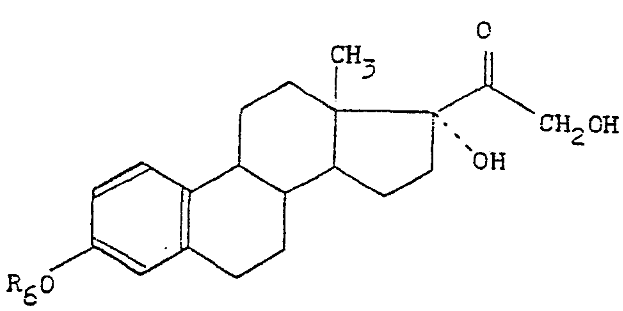

(I")

worin $R_6$ die in Anspruch 3 angegebene Bedeutung besitzt, dadurch gekennzeichnet, daß man das in den Ansprüchen 1 bis 3 beschriebene Verfahren, ausgehend von den Produkten der Formel (II'), durchführt, worin die Ringe $A'_2$ und $B'_2$ die folgenden sind :

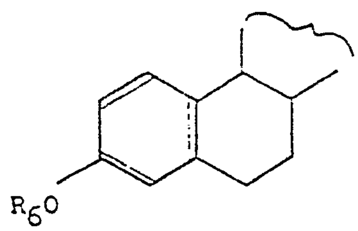

5. Verfahren gemäß einem der Ansprüche 1 bis 4, dadurch gekennzeichnet, daß das Reagens zur Einführung des Acetoxylrestes Bleitetraacetat ist.

6. Verfahren gemäß Anspruch 3 oder 4, dadurch gekennzeichnet, daß der Rest $R_6$ einen Methylrest bedeutet und die Reste R' und R" gemeinsam einen Ethylenrest bilden.

7. Als neue, industrielle Produkte die Produkte der Formel ($IV'_A$)

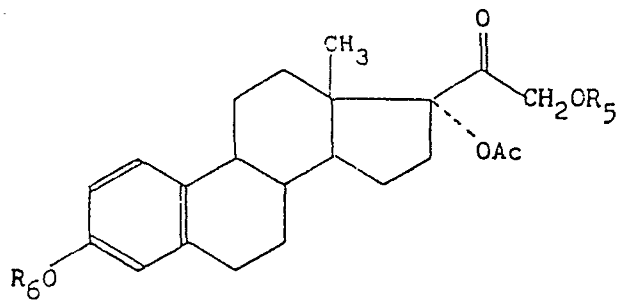

($IV'_A$)

worin $R_5$ und $R_6$ die in den Ansprüchen 1 und 3 angegebenen Bedeutungen besitzen.

**Ansprüche** (für den Vertragsstaat AT)

1. Verfahren zur Herstellung der Produkte der Formel (I)

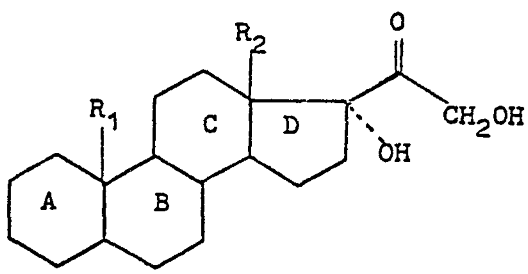

(I)

worin $R_1$ ein Wasserstoffatom, einen Alkylrest mit 1 bis 4 Kohlenstoffatomen, der gegebenenfalls durch eine Sauerstoff oder Stockstoff enthaltende Funktion oder durch ein Halogenatom substituiert ist, bedeutet oder $R_1$ einen Alkenyl- oder Alkinylrest mit 2 bis 4 Kohlenstoffatomen darstellt, $R_2$ einen Alkylrest mit 1 bis 4 Kohlenstoffatomen bedeutet, die Ringe A, B, C und D gegebenenfalls eine oder mehrere Doppelbindungen enthalten und gegebenenfalls durch eine oder mehrere Hydroxyl- oder Ketonfunktionen, durch ein oder mehrere Halogenatome, durch einen oder mehrere Alkyl- oder Alkoxyreste mit 1 bis 4 Kohlenstoffatomen oder durch einen oder mehrere Alkenyl- oder Alkinylreste mit 2 bis 4 Kohlenstoffatomen substituiert sind, dadurch gekennzeichnet, daß man ein Produkt der Formel (II)

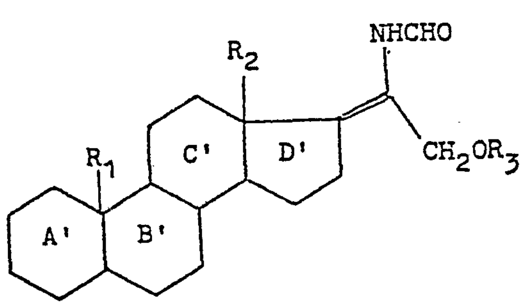

(II)

worin $R_1$ und $R_2$ die vorstehend angegebene Bedeutung besitzen und die Ringe A', B', C' und D' die vorstehend für A, B, C und D angegebenen Bedeutungen mit der zusätzlichen Möglichkeit besitzen, durch geschützte Hydroxyl- oder Ketonfunktionen substituiert zu sein, und $R_3$ ein Wasserstoffatom oder $R'_3$ bedeutet, wobei $R'_3$ eine Schutzgruppe für den Hydroxylrest darstellt, mit einem Reagens zur Einführung eines Acetoxylrestes behandelt, um ein Produkt der Formel (III)

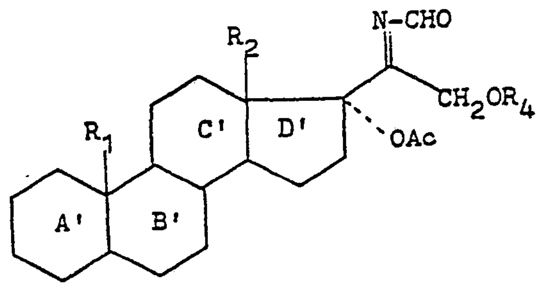

(III)

zu erhalten, worin $R_4$ für $R'_3$ oder einen Acetylrest steht und Ac einen Acetylrest bedeutet, welches Produkt man mit einem Hydrolysemittel behandelt, um ein Produkt der Formel (IV)

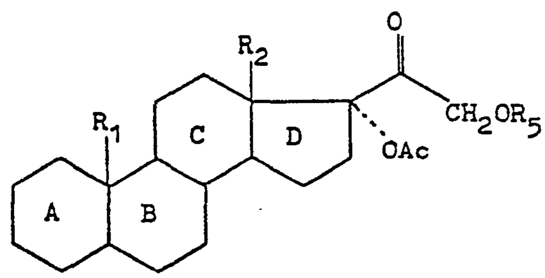

(IV)

zu erhalten, worin $R_1$, $R_2$, Ac, A, B, C und D die vorstehend angegebene Bedeutung besitzen und $R_5$ ein Wasserstoffatom, eine nicht leicht hydrolysierbare Schutzgruppe für den Hydroxylrest oder einen Acetylrest bedeutet, welches Produkt man zur Entfernung des Acetylrestes in 17α-Stellung und der Gruppe $R_5$, wenn diese kein Wasserstoffatom bedeutet, behandelt, um ein Produkt der Formel (I) zu erhalten.

2. Verfahren gemäß Anspruch 1, dadurch gekennzeichnet, daß man bei dem Verfahren von Produkten der Formel (II) ausgeht, worin $R_2$ einen Methylrest bedeutet und $R_1$ ein Wasserstoffatom oder einen Methyl- oder Ethinylrest bedeutet.

3. Verfahren gemäß den Ansprüchen 1 oder 2 zur Herstellung der Produkte der Formel (I')

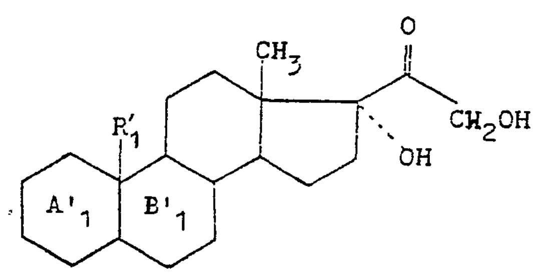

(I')

worin $R'_1$, $A'_1$ und $B'_1$ derart sind, daß die Ringe $A'_1$ und $B'_1$ ausgewählt sind aus der Gruppe der folgenden Systeme :

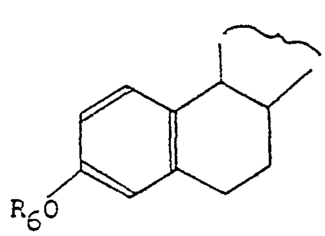

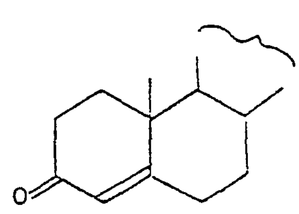

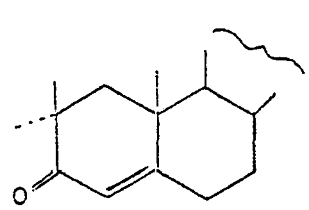

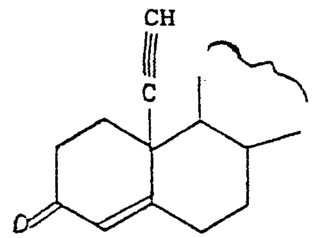

worin $R_6$ einen Alkylrest mit 1 bis 8 Kohlenstoffatomen oder einen Aralkylrest mit 7 bis 12 Kohlenstoffatomen bedeutet, dadurch gekennzeichnet, daß man ein Produkt der Formel (II')

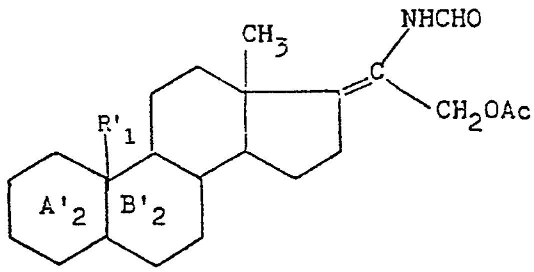

(II')

worin $R'_1$ die vorstehend angegebene Bedeutung besitzt, Ac eine Acetylrest bedeutet und die Ringe $A'_2$ und $B'_2$ ausgewählt sind aus der Gruppe der folgenden Systeme :

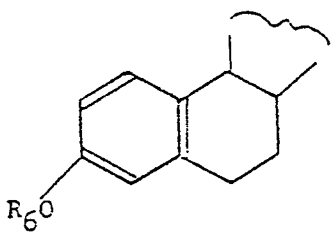

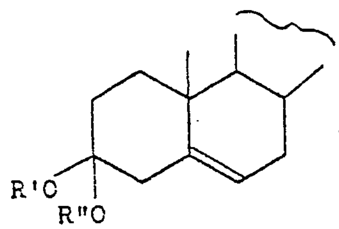

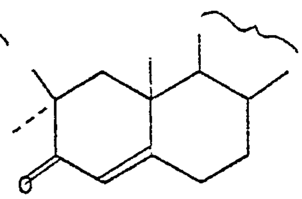

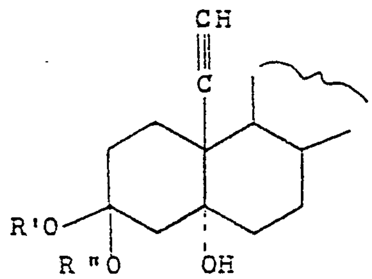

worin R' und R'', die gleich oder verschieden sind einen Methyl- oder Ethylrest bedeuten oder R' und R'' gemeinsam einen Ethylen- oder Trimethylenrest bilden, mit einem Reagens zur Einführung eines Acetoxylrestes umsetzt, um ein Produkt der Formel (III')

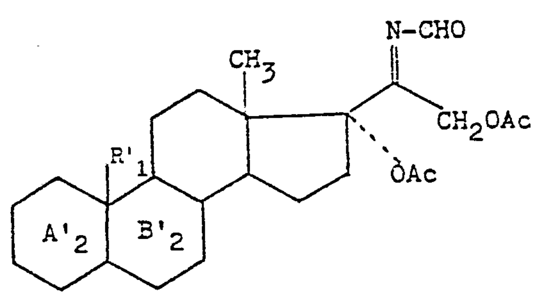

(III')

zu erhalten, worin $R'_1$, Ac, $A'_2$ und $B'_2$ die vorstehende Bedeutung besitzen, welches Produkt man mit einem Hydrolysemittel behandelt, um ein Produkt der Formel (IV')

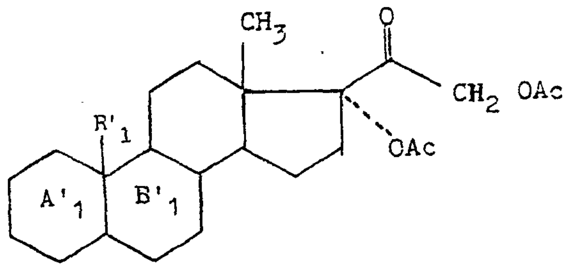

(IV')

zu erhalten, worin $R'_1$, $A'_1$ und $B'_1$ die vorstehende Bedeutung besitzen, welches Produkt man verseift, um ein gewünschtes Produkt der Formel (I') zu erhalten.

4. Verfahren gemäß den Ansprüchen 1 bis 3 zur Herstellung der Produkte der Formel (I'')

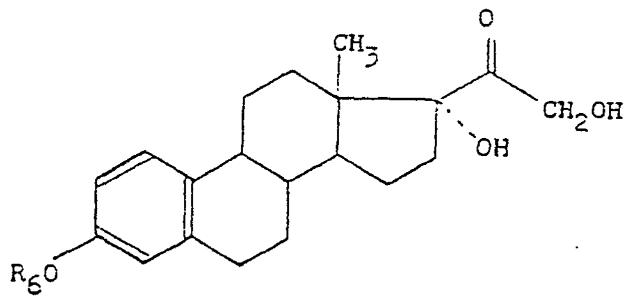

(I'')

worin $R_6$ die in Anspruch 3 angegebene Bedeutung besitzt, dadurch gekennzeichnet, daß man das Verfahren gemäß den Ansprüchen 1 bis 3, ausgehend von den Produkten der Formel (II'), durchführt, worin die Ringe $A'_2$ und $B'_2$ die folgenden sind :

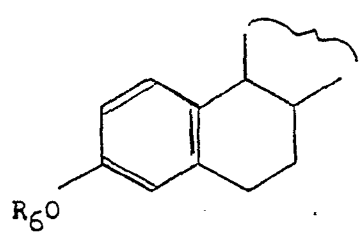

5. Verfahren gemäß einem der Ansprüche 1 bis 4, dadurch gekennzeichnet, daß das Reagens zur Einführung des Acetoxylrestes Bleitetraacetat ist.

6. Verfahren gemäß den Ansprüchen 3 oder 4, dadurch gekennzeichnet, daß der Rest $R_6$ einen Methylrest bedeutet und die Reste R' und R'' gemeinsam einen Ethylenrest bilden.